# EUROPÄISCHE PATENTSCHRIFT

(11) **EP 2 658 450 B1**
(45) Veröffentlichungstag und Bekanntmachung des Hinweises auf die Patenterteilung: **21.01.2015**
(21) Anmeldenummer: 11801752.4
(22) Anmeldetag: 23.12.2011
(51) Int. Cl.: A61B 5/151, A61B 5/157

(54) **BEHÄLTER AUS EINEM ALUMINIUMFOLIEN-POLYMER-VERBUND FÜR ANALYTISCHE HILFSMITTEL UND VERFAHREN ZU DESSEN HERSTELLTUNG**
CONTAINER MADE FROM A COMPOSITE OF ALUMINIUM FOIL AND POLYMER AND USED FOR ANALYTICAL AIDS, AND METHOD FOR PRODUCING SAID CONTAINER
RÉCIPIENT À BASE DE COMPOSITE FEUILLES D'ALUMINIUM - POLYMÈRE POUR AGENTS ANALYTIQUES ET PROCÉDÉ DE PRODUCTION DUDIT RÉCIPIENT

(30) Priorität: 27.12.2010 EP 10016099; 27.12.2010 EP 10016100; 13.04.2011 EP 11003111
(43) Veröffentlichungstag der Anmeldung: 06.11.2013
(73) Patentinhaber: Roche Diagnostics GmbH, 68305 Mannheim (DE); F.Hoffmann-La Roche AG, 4070 Basel (CH)
(72) Erfinder: LEICHNER, Wilhelm, 68307 Mannheim (DE)
(74) Vertreter: Stößel, Matthias
(86) Internationale Anmeldenummer: PCT/EP2011/073940
(87) Internationale Veröffentlichungsnummer: WO 2012/089660

(56) Entgegenhaltungen:
- EP-A1- 1 360 935
- EP-A1- 1 813 220
- DE-A1- 19 854 316
- US-A1- 2009 257 911

## Beschreibung

### Gebiet der Erfindung

Die Erfindung liegt auf dem Gebiet der Behälter bzw. Verpackungen für analytische Hilfsmittel, insbesondere für Magazine, im Speziellen der Magazine für analytische Sensoren zur Messung von Körperflüssigkeitsparametern beispielsweise in einer Körperflüssigkeit eines Benutzers. Die Erfindung betrifft ein Verfahren zur Herstellung eines Behälters, insbesondere ausgestaltet, um ein analytisches Hilfsmittel, im Besonderen hydrophiler Sensoren, aufzunehmen und deren sterilen Verpackung zu erlauben.

### Stand der Technik

Aus dem Stand der Technik sind Behälter oder Verpackungen, auch als Magazine bezeichnet, von Sensoren oder anderen steril zu haltenden Gegenständen bekannt. Hierbei liegt das besondere Augenmerk auf der Gewährleistung der Sterilität solcher Gegenstände über einen möglichst langen Zeitraum. Die Sterilität ist besonders wichtig für Gegenstände, die in den Körper eines Menschen eindringen sollen, wie analytische Hilfsmittel.

In der Lebensmittelverpackung werden häufig Metallverbunde, besonders häufig aluminiumhaltige Verbunde verwendet, um einerseits eine lebensmittelhygienische Verpackung bereitzustellen, darüber hinaus aber nicht zu schwere Verpackungen zu generieren, die gleichwohl ausreichende stabil, insbesondere stapelbar, und luftdicht sind.

Solche Verpackungen sind beispielsweise aus den Dokumenten WO 2007/029755 oder EP-A-1 640 277 bekannt, wobei ein thermoplastisches Material auf einer Aluminiumoberfläche aufgebracht wird, um eine ausreichende Stabilität für die Bevorratung von Kohlensäure haltigen Lebensmittels zu bewerkstelligen. Diese Behältnisse gewährleisten zwar eine ausreichende Stabilität zur Handhabung der Lebensmittel im Alltag, weisen dabei jedoch den Nachteil auf, dass sie eine Mindestdicke aufweisen müssen, um die Stabilität für die Handhabung durch einen Benutzer zu gewährleisten und den Beanspruchungen durch die direkte Einwirkung des Lebensmittels auf der einen Seite und den Beanspruchungen durch die Umwelt, wie bei Transporten, zu genügen.

Weitere Verpackungen aus dem medizinischen Bereich sind bekannt, die ein hermetisches Verpacken von steril zu haltenden Gegenständen gewährleisten. So werden häufig steril zu verpackende Medikamente in Aluminiumblistern verpackt, wie in der NL-A-1023464 beschrieben, wobei ebenfalls aufgrund der Handhabung der Blister im Alltag durch zum Teil motorisch eingeschränkte Patienten eine ausreichende Stabilität gewährleistet sein muss.

In EP 1 360 935 A1 wird eine Verpackung zum Halten von Testern zum Zugreifen, Sammeln und Analysieren einer Probe eines Fluids beschrieben. Die Verpackung umfasst einen ersten Streifenabschnitt mit einer Längsdimension und einer Breitendimension und mit mehreren seriell ausgerichteten Vertiefungen, von denen jede eine Öffnung zum Aufnehmen eines Testers umfasst und konfiguriert ist, den Tester hierin zu halten. Die Verpackung umfasst weiterhin einen zweiten Streifenabschnitt mit Längen- und Breitendimensionen ähnlich den Längen- und Breitendimensionen des ersten Streifenabschnitts, wobei der zweite Streifenabschnitt die Öffnung jeder Vertiefung bedeckt. Die Verpackung umfasst weiterhin Mittel zum Anbringen eines Testers relativ zu jeder der zugehörigen Vertiefungen.

In der WO 2010/094426 wird die Herstellung und Verpackung eines medizinischen Hilfsmittels beschrieben, wobei ebenfalls Aluminium haltige Materialien zur Sterilhaltung der medizinischen Hilfsmittel beschrieben werden. Es wird darüber hinaus ein Laserschweißverfahren beschrieben, um medizinische Hilfsmittel steril in solchen Verpackungen zu verschließen. Eine nähere Beschreibung der Beschaffenheit der Materialien und deren Verarbeitung werden hier jedoch nicht angegeben.

Besonders bei der Bereitstellung integrierter analytischer Hilfsmittel, wie in der WO 2010/094426 beschrieben, die sowohl für die Bereitstellung von Körperflüssigkeit eingesetzt als auch zu deren Analyse verwendet werden, ist es wichtig eine geeignete Bevorratung dieser hochempfindlichen Hilfsmittel zu gewährleisten. So ist es einerseits wichtig eine Verpackung zu wählen, die es ermöglicht eine ausreichende Sterilität der analytischen Hilfsmittel zu gewährleisten, darüber hinaus aber auch zu verhindern, dass die hochempfindlichen Oberflächen der analytischen Hilfsmittel durch die Verpackung beeinträchtigt werden.

### Aufgabe der Erfindung

Es ist daher eine Aufgabe der vorliegenden Erfindung, mindestens einen der sich aus dem Stand der Technik ergebenden Nachteile mindestens teilweise zu überwinden. Insbesondere soll ein Behälter vorgeschlagen werden, der eine möglichst einfache und effiziente Bevorratung von analytischen Hilfsmitteln gewährleistet.

So sollen die analytischen Hilfsmittel einerseits geschützt, andererseits leicht zugänglich verpackt werden, um sowohl eine lange Haltbarkeit und Funktionsfähigkeit der Hilfsmittel zu gewährleisten als auch eine geringe Beeinträchtigung bei Benutzung. Darüber hinaus soll ein Verfahren zur Herstellung solch eines Behälters vorgeschlagen werden.

### Offenbarung der Erfindung

Einen Beitrag zur Lösung mindestens einer der vorstehenden Aufgaben leistet die Erfindung mit den Merkmalen der unabhängigen Patentansprüche. Vorteilhafte Weiterbildungen der Erfindung, welche einzeln oder in beliebiger Kombination realisierbar sind, sind in den abhängigen Patentansprüchen dargestellt.

Ein erster Aspekt der vorliegenden Erfindung betrifft einen Behälter zumindest teilweise, vorzugsweise zu mindestens 10 %, vorzugsweise zu mindestens 20 %, vorzugsweise zu mindestens 30 %, vorzugsweise mindestens 50 % und besonders bevorzugt mindestens 70 % der Außenfläche, jeweils bezogen auf die Gesamtaußenfläche des Behälters, gebildet aus einem flächigen Verbundmaterial, wobei das Verbundmaterial beinhaltet:
- eine Aluminiumfolie mit einer ersten und einer zweiten Oberflächenseite,
- eine erste Polymerschicht, die mit mindestens einer der beiden Oberflächenseiten verbunden ist,
wobei die Aluminiumfolie über die Polymerschicht mindestens eine Öffnung einer Fassung bedeckt, wobei das Verbundmaterial zusammen mit der Fassung den Behälter bildet, wobei die Fassung mindestens ein analytisches Hilfsmittel in einer Ausnehmung aufnimmt, wobei die Aluminiumfolie gepresst oder tiefgezogen ist.

In einer bevorzugten Ausgestaltung des Behälters sind 5 bis 70 %, bevorzugt 10 bis 50 %, besonders bevorzugt 15 bis 30 % der Gesamtaußenfläche des Behälters mit dem flächigen Verbundmaterial bedeckt. Besonders bevorzugt ist das Verbundmaterial radial an der Außenfläche des Behälters angeordnet.

Der Behälter umgibt mindestens ein analytisches Hilfsmittel, vorzugsweise als Verpackung, um das analytische Hilfsmittel von der Umgebung abzutrennen. Von Abtrennen ist bevorzugt die Rede, wenn die Verpackung das analytische Hilfsmittel von allen Seiten umgibt und somit ganz von der Umgebung abtrennt, um so beispielsweise eine sterile Verpackung zu gewährleisten. Der Behälter wird zu mindestens einem Teil durch eine Fassung gebildet, die das mindestens eine analytische Hilfsmittel in mindestens einer Ausnehmung aufnimmt. Die Fassung hat die Funktion mindestens ein analytisches Hilfsmittel zumindest zu einem Teil zu umgeben und es so vor seiner Umgebung zu schützen. Dabei soll die Fassung das analytische Hilfsmittel zum Einen vor mechanischen Einflüssen schützen, zum Anderen das analytische Hilfsmittel steril halten können. Darüber hinaus sollte die Fassung eine Möglichkeit bieten, auch bei Bevorratung von mehreren analytischen Hilfsmitteln diese gut transportieren zu können und für den Benutzer oder gegebenenfalls für eine Benutzungsvorrichtung leicht zugänglich zu machen.

Die Fassung kann verschiedene Formen aufweisen. Beispielsweise kann die Fassung eine quaderförmige, zylindrische oder kugelförmige, insbesondere kreisförmige bzw. discförmige Form aufweisen. Die Fassung weist bevorzugt mindestens ein Grundelement, beispielsweise in Form eines Rahmens zur Bevorratung von analytischen Hilfsmitteln auf. Der Rahmen kann zur einfacheren Handhabung der Fassung und ihrer Stabilisierung dienen. In oder an dem Rahmen der Fassung befindet sich mindestens eine Ausnehmung zur Aufnahme mindestens eines analytischen Hilfsmittels. Wie später noch detailliert ausgeführt, können sich mehrere Ausnehmungen in der Fassung befinden. Diese Ausnehmungen können ein oder mehrere analytische Hilfsmittel aufnehmen oder sonstige Hilfsmittel, die zur Benutzung der Fassung hilfreich sind. Diese mindestens eine Ausnehmung dient dazu das mindestens eine analytische Hilfsmittel mindestens zu einem Teil zu umgeben, um es für seinen späteren Gebrauch geschützt, bevorzugt steril, aufbewahren zu können. Die Ausnehmung kann mehrere Wandungen aufweisen, die das analytische Hilfsmittel umgeben. Die Wandungen können dabei mindestens einen Teil eines Raums umschließen in den das analytische Hilfsmittel eingebracht wird. Der Raum kann dabei alle denkbaren Formen annehmen, solange er geeignet ist, das analytische Hilfsmittel aufzunehmen und für seinen Gebrauch zugänglich zu machen. Bevorzugt weist die mindestens eine Ausnehmung eine längliche Form, angepasst an die längliche Form des bevorzugten analytischen Hilfsmittels, auf. Die mindestens eine Ausnehmung kann beispielsweise kugelförmig, zylindrisch oder, wie bevorzugt, quaderförmig ausgestaltet sein. Zur Befüllung der Ausnehmung mit analytischen Hilfsmitteln sollte die Ausnehmung wenigstens eine Öffnung aufweisen. Diese Öffnung kann an jeder beliebigen, bevorzugt von außen zugänglichen, Seite der Ausnehmung eingebracht sein. Bevorzugter Weise weist die Fassung mindestens zwei Öffnungen auf, die sich bevorzugt in oder angrenzend zu der mindestens einen Ausnehmung befinden. Besonders bevorzugt weist die Ausnehmung an den Querseiten des Quaders eine Öffnung auf. Alternativ können sich die Öffnungen auch an einer länglichen Seite und an einer Querseite befinden, oder alternativ nur an den länglichen Seiten. Hierbei können die Öffnungen verschiedenen Zwecken dienen. So kann beispielsweise die längliche Öffnung dazu dienen das analytische Hilfsmittel in die Ausnehmung und damit in den späteren Behälter einzufügen. Die Öffnung bzw. Öffnungen in der Querseite können beispielsweise zur Verwendung des analytischen Hilfsmittels bei seinem späteren Gebrauch dienen. So kann eine Öffnung dazu ausgestaltet sein, das analytische Hilfsmittel bereitzustellen und eine weitere Öffnung dazu dienen ein Element einer Gebrauchsvorrichtung aufzunehmen, um das analytische Hilfsmittel in eine Gebrauchsposition zu führen. Andere Ausgestaltungen sind jedoch auch denkbar.

Befinden sich mehr als eine Ausnehmung in der Fassung, so können diese bevorzugt nebeneinander in dem Rahmen der Fassung angeordnet sein. Ausgestaltungen der Fassung mit Ausnehmungen übereinander oder hintereinander sind ebenfalls denkbar. Bevorzugt sind die Ausnehmungen kreisförmig nebeneinander angeordnet. Besonders bevorzugt weisen die Ausnehmungen Öffnungen parallel zur Kreisfläche und quer zur Kreisfläche auf. In einer bevorzugten Ausgestaltung sind die Ausnehmungen länglich, in Form eines Quaders ausgestaltet, wobei sie mit ihrer länglichen Ausdehnung strahlenförmig an der Außenseite der discförmigen Fassung angeordnet sind.

Das analytische Hilfsmittel ist bevorzugt so in der Ausnehmung gelagert, dass es möglichst wenig Kontakt mit dem Rahmen bzw. den Wandungen der Ausnehmung hat. Dies ist besonders dann bevorzugt, wenn das analytische Hilfsmittel Bereiche aufweist, die vor ihrer Benutzung vor Kontakt mit anderen Gegenständen, Flüssigkeiten oder sonstigen Umgebungen geschützt werden sollen. Das analytische Hilfsmittel kann dazu einen Bereich aufweisen, der zur Kontaktierung mit der Fassung dient und es ermöglicht das analytische Hilfsmittel ohne weitere Beeinflussung in der Ausnehmung zu lagern.

Die Fassung kann aus verschiedenen Materialien hergestellt sein. Bevorzugt werden Materialien verwendet, die zum Einen eine ausreichende mechanische Stabilität aufweisen, um vor Stößen zu schützen und von einem Benutzer gehandhabt zu werden und dabei nicht zu schwer sind, um möglichst viele analytische Hilfsmittel bevorraten zu können. Darüber hinaus sollte das Material der Fassung bzw. des Behälters eine gute Beständigkeit gegen energiereiche Strahlung aufweisen (beispielsweise beta-Strahlung, Gamma- oder Röntgenstrahlung), wenn die analytischen Hilfsmittel steril gehalten werden sollen. Es sollte gewährleistet sein, dass auch nach Bestrahlung eine ausreichende Stabilität des Behälters für den geplanten Nutzungszeitraum besteht und eine ausreichende Dichtigkeit gegenüber Keimen für diesen Zeitraum. Je nach Anwendung des analytischen Hilfsmittels kann dies mehrere Jahre sein. Die Fassung kann folglich aus jedem Material sein, das der Fachmann kennt, um diese Eigenschaften zu erreichen. So kann die Fassung beispielsweise aus einem Blech, zum Beispiel einem Stahl- oder Aluminiumblech gefertigt sein. Die Fassung kann zusätzlich oder alternativ aus einem Kunststoff gefertigt sein, wie beispielsweise einem Polycarbonat (PC) oder einem Copolyester, wie beispielsweise Polyphenylsulfon (PPSU). Es sind auch Verbundmaterialien aus Metall und Kunststoffen denkbar.

Zum Verschließen der Öffnungen der Fassung bzw. des Behälters wird eine Aluminiumfolie verwendet, die zumindest mit einer Polymerschicht ein flächiges Verbundmaterial bildet.

Unter einer Aluminiumfolie ist ein Aluminium haltiges Gebilde zu verstehen, das eine deutlich größere Ausdehnung in eine erste, auch Länge der Aluminiumfolie genannt, und eine zweite Raumrichtungen, auch Breite der Folie genannt, hat als in eine dritte Raumrichtung, auch Dicke genannt. Die Aluminium haltige Folie kann noch weitere Bestandteile enthalten, wie beispielsweise weitere Metalle, wie Eisen, Kupfer, Gold, Silber oder Nichtmetallische Materialien oder Mischungen daraus. Sie kann darüber hinaus auch andere oder weitere Materialien enthalten, wie beispielsweise Pigmente, beispielsweise in Form von Farbstoffen oder beispielsweise Füllstoffe, beispielsweise in Form von organischen Verbindungen. Bevorzugt besteht die Aluminiumfolie zu mindestens 95, vorzugsweise 97 und besonders bevorzugt 99 Gew.-%, jeweils bezogen auf die Aluminiumfolie, aus Aluminium. Bevorzugt besteht die Aluminiumfolie zu 90 bis 100 Gew.-%, besonders bevorzugt zu 95 bis 100 Gew.-%, ganz besonders bevorzugt zu 97 bis 100 Gew.-% aus Aluminium.

Die Ausdehnung der Folie kann in eine erste und eine zweite Raumrichtung mehrere Meter betragen, während sie in die dritte Raumrichtung, auch Dicke oder Querschnitt genannt, beispielsweise nur wenige mm oder µm betragen kann. Bevorzugter Weise weist die Aluminiumfolie eine einheitliche Dicke auf. So liegt beispielsweise die Abweichung des Querschnitts einer solchen Aluminiumfolie bei wenigen µm, bevorzugt zwischen 0,1 und 10 µm, besonders bevorzugt zwischen 0,1 und 3 µm.

Erfindungsgemäß ist der Behälter erhältlich, in dem auf mindestens einer Seite der Aluminiumfolie eine erste Polymerschicht vorgesehen ist. Die Polymerschicht kann auf verschiedene Weisen auf der Aluminiumfolie oder benachbart zur Aluminiumfolie vorgesehen werden. So kann beispielsweise die Polymerschicht eine Polymerfolie sein, die mit der Aluminiumfolie verklebt wird. Das Verkleben kann beispielsweise durch Aufschmelzen der Polymerfolie erfolgen, wobei die Polymerfolie zunächst mit der Aluminiumfolie in Kontakt gebracht wird und dann beides erhitzt und zusammengedrückt wird. Es können sich alternativ noch weitere Schichten zwischen der Aluminiumfolie und der Polymerschicht befinden. So kann beispielsweise eine Klebeschicht zwischen der Aluminiumfolie und der Polymerschicht angeordnet sein, die dazu dient die Folie mit der Schicht zu verbinden. Genauso kann die Polymerschicht oder auch die Polymerfolie klebend ausgerüstet sein. Dieses wird meist durch Co-Polymerisieren von funktionellen Monomeren erreicht, die über funktionelle Gruppen verfügen, die mit der Aluminiumfolienoberfläche chemisch reagieren können. Wird ein flächiges Verbundmaterial eingesetzt, das vor Bildung des Behälters gepresst oder tiefgezogen wurde, so kann die Polymerschicht alternativ vor dem Pressen oder Tiefziehen der Aluminiumfolie oder nach dem Formen der Aluminiumfolie vorgesehen werden.

Bevorzugt wird die Aluminiumfolie oder das flächige Verbundmaterial vor Bilden des Behälters gepresst oder tiefgezogen. Weiterhin bevorzugt wird mindestens die erste Polymerschicht vor dem Formen der Aluminiumfolie mit der Aluminiumfolie verbunden.

Das Formen stellt vorzugsweise eine Festlegung des Verlaufs des flächigen Verbundmaterials bzw. der Aluminiumfolie an der verformten Stelle dar. Es kann durch das Formen beispielsweise ein Falz oder ein Knick in das sonst flächige Verbundmaterial bzw. in die Aluminiumfolie eingebracht werden. Durch das Verformen kann eine Fixierung des Verlaufs dies- und jenseits dieses Knicks bewirkt werden. Bevorzugt findet bei dem Formen ein Abknicken des flächigen Verbundmaterials bzw. der Aluminiumfolie statt, das eine Verlaufsänderung der Aluminiumfolie in einem Bereich zwischen 10° und 170°, bevorzugt in einem Bereich zwischen 30° und 150°, besonders bevorzugt in einem Bereich zwischen 50° und 120° bewirkt. Der Knick stellt bevorzugt eine Unstetigkeit in dem Verlauf der Aluminiumfolie bzw. des Verbundmaterials dar, der zwei Bereiche der Aluminiumfolie bzw. des Verbundmaterials in einer abgewinkelten Anordnung zueinander fixiert. An dieser Knickstelle kann die Aluminiumfolie bzw. das Verbundmaterial bevorzugt nicht flexibel gebogen werden, wie dies in den nicht geknickten Bereichen weiterhin möglich ist.

Weiterhin können an verschiedenen Stellen der Aluminiumfolie oder des flächigen Verbundmaterials unterschiedlich starke Verformungen vorgenommen werden. So kann die Aluminiumfolie oder das flächige Verbundmaterial an einer Stelle um 120° und an einer anderen Stelle um 90° verformt werden. Dies kann beispielsweise beim Pressen bzw. beim Tiefziehen durch die Wahl des verwendeten Stempels, wie später noch erläutert, bewirkt werden. Aufgrund des Formens kann die Aluminiumfolie oder das flächige Verbundmaterial beispielsweise an mehr als einer Stelle der Fassung mit dieser in Kontakt treten. Auf diese Weise können beispielsweise Öffnungen, die sich über mehr als eine Oberfläche erstrecken oder mehrere Öffnungen, die sich in verschiedenen Oberflächen der Fassung befinden, mit nur einer Aluminiumfolie bzw. einem flächigen Verbundmaterial, abgedeckt werden. Der Vorgang des Tiefziehens bzw. Pressens wird später beim Verfahren zur Herstellung des Behälters noch näher erläutert.

Zu diesem Formen der Aluminiumfolie kann darüber hinaus vor dem Formen ein Prägen stattfinden, wie es später noch näher erläutert wird. Das Aufbringen der Polymerschicht auf oder an die Aluminiumfolie kann auch hier alternativ vor oder nach dem Prägen erfolgen.

Allgemein ist es in einer erfindungsgemäßen Ausgestaltung bevorzugt, dass die erste Polymerschicht aus einem thermoplastischen Polymer besteht. Die erste Polymerschicht kann neben dem thermoplastischen Polymer noch weitere Additive, meist weniger als 35 Gew.-%, vorzugsweise weniger als 15 Gew.-% und besonders bevorzugt weniger als 7 Gew.-%, jeweils bezogen auf die erste Polymerschicht, enthalten. Derartige Additive können Pigmente, Antistatikmittel und andere sein, die häufig mit thermoplastischen Polymeren verarbeitet werden, um deren Eigenschaften an eine bestimmte Anwendung anzupassen. Ferner kann das erste thermoplastische Polymer auch aus einer Mischung aus zwei oder mehr von einander verschiedenen thermoplastischen Polymeren bestehen. Zudem ist es bevorzugt, dass die erfindungsgemäß eingesetzten thermoplastischen Polymere eine Schmelztemperatur von mehr als 50°C, vorzugsweise mehr als 90° C und besonders bevorzugt mehr als 130°C aufweisen. Bevorzugt handelt es sich bei dem thermoplastischen Polymer um einen Polyester. Beispiele solcher Harze sind handelsübliche Produkte sowie deren Gemische, wie Vitel^{®}2100B, Vitel^{®}2200B, Vitel^{®}3200B und Vitel^{®}3300B.

Besonders bevorzugt handelt es sich bei dem thermoplastischen Polymer um geradlinige, gesättigte Copolymere verschiedener Polyester. Bevorzugt weist das thermoplastische Polymer weitere Additive in einem Bereich von 0 bis 50 Gew.-%, bevorzugt in einem Bereich von 5 bis 30 Gew.-%, besonders bevorzugt in einem Bereich von 10 bis 20 Gew.-% auf. Als Additive können beispielsweise Schmierstoffe eingesetzt werden. Beispiele für Additive sind ausgewählt aus der Gruppe bestehend aus Molybdänsulfid (MoS₂), Teflon, Graphit (C), Kupfer (Cu), Blei (Pb), Keramiken und Kunststoffe oder mindestens zwei davon.

Alternativ oder zusätzlich kann die Polymerschicht hydrophil ausgebildet sein. Weiterhin kann eine zusätzliche Polymerschicht verwendet werden, die hydrophil ausgestaltet ist. Die Hydrophilie eines Polymers kann durch die Wahl seiner Substituenten beeinflusst werden. So bewirken eine Erhöhung des Anteils an hydrophilen Substituenten eine Erhöhung der Hydrophilie des Polymers. Als hydrophile Substituenten werden bevorzugt Substituenten verwendet, ausgewählt aus der Gruppe bestehend aus Hydroxygruppe (-OH), Schwefelwasserstoffgruppe (-SH), Amingruppe (-NH₂, -NRH), und Säuregruppen (-OOH), sowie mindestens zwei davon. Bevorzugt weisen die Polymere zum Aufbau der Polymerschicht einen Anteil an hydrophilen Substituenten von 10 bis 80 Gew.-%, bevorzugt von 20 bis 70 Gew.-%, besonders bevorzugt von 30 bis 60 Gew.-% auf.

Allgemein kann die erste und die weitere Polymerschicht durch jede dem Fachmann bekannte und geeignet erscheinende Methode auf die Aluminiumfolie gebracht werden. So kann das Polymer als Schmelze, als Lösung in einem Lösemittel oder aus einer Kombination aus Schmelze und Lösung aufgebracht werden. Als Methoden kommen beispielsweise Sprühen, Spin-Coaten, Rakeln oder Streichen oder eine Kombination aus mindestens zwei dieser Methoden in Betracht. Erfindungsgemäß ist es bevorzugt, dass das Vorsehen der ersten Polymerschicht oder das Vorsehen der weiteren Polymerschicht ein Aufrakeln eines in Lösung gebrachten Polymeres ist. Hierzu können dem Fachmann bekannte Rakel und Lösungsmittel verwendet werden. Alternativ kann das Aufbringen der Polymerschicht ein Aufschmelzen und Erstarren der ersten Polymerschicht oder der weiteren Polymerschicht oder beider beinhaltet. Hierdurch kann vorzugsweise ein Eindringen der Polymerschmelze in die Kavitäten eines Substrats, hier in der Regel die Oberfläche der Aluminiumfolie, erreicht werden. Durch das Erstarren der Polymerschmelze in den Kavitäten wird die Polymerschicht auf und in dem Substrat verankert. Dieses wird oft als Siegeln oder Heißsiegeln bezeichnet. Ein derartiger Prozess kann zum Verbinden der beschichteten Aluminiumfolie mit weiteren Materialien, wie der Fassung zur Bildung eines Behälters in Form eines Magazins benutzt werden. Das Heißsiegeln wird bevorzugt bei einer Temperatur zwischen 160 und 280°C, vorzugsweise zwischen 200 und 250°C vorgenommen. Die Wahl der Temperatur hängt von der Zusammensetzung der aufzuschmelzenden Materialien ab. So weisen viele Kunststoffe eine Schmelztemperatur zwischen 220 und 240°C auf. Alternativ oder ergänzend dazu können funktionelle Gruppen in dem thermoplastischen Polymer vorgesehen sein, die mit der Substratoberfläche chemische Bindungen eingehen können. Dieses wird oft als Kleben bezeichnet. Erfindungsgemäß kann als Verbinden sowohl das Siegeln, Kleben oder eine Kombination aus Siegeln und Kleben erfolgen. Hierbei ist es bevorzugt, dass das Verbinden möglichst Lösemittelarm, oder gar Lösemittelfrei, erfolgt. So ist es erfindungsgemäß bevorzugt, beim Verbinden weniger als 10 Gew.-%, vorzugsweise weniger als 1 Gew.-% und besonders bevorzugt weniger als 0,1 Gew.-%, jeweils bezogen auf das thermoplastische Polymer, eines Lösemittels einzusetzen. Ferner ist es erfindungsgemäß bevorzugt, bei dem Verbinden möglichst wenig, vorzugsweise weniger als 10 Gew.-%, vorzugsweise weniger als 1 Gew.-% und besonders bevorzugt weniger als 0,1 Gew.-%, jeweils bezogen auf das thermoplastische Polymer, oder gar keinen niedermolekularen Kleber, der bereits bei 30°C flüssig ist, einzusetzen. Damit beinhaltet der erfindungsgemäße Behälter in einer Ausgestaltung ein Verbundmaterial, das weniger als 1 Gew.-%, vorzugsweise weniger als 0,1 Gew.-% und besonders bevorzugt weniger als 0,01 Gew.-%, jeweils bezogen auf das thermoplastische Polymer des Verbundmaterials, ein Lösemittel enthält. Weiterhin beinhaltet der erfindungsgemäße Behälter in einer anderen Ausgestaltung ein Verbundmaterial, das weniger als 1 Gew.-%, vorzugsweise weniger als 0,1 Gew.-% und besonders bevorzugt weniger als 0,01 Gew.-%, jeweils bezogen auf das thermoplastische Polymer des Verbundmaterials, einen niedermolekularen Kleber enthält.

Weiterhin ist es bevorzugt beim Verbinden ein Lösemittel in einem Bereich von 0,01 bis 10 Gew.-%, vorzugsweise in einem Bereich von 0,01 bis 1 Gew.-% und besonders bevorzugt in einem Bereich von 0,01 bis 0,1 Gew.-%, jeweils bezogen auf das thermoplastische Polymer, einzusetzen. Ferner ist es erfindungsgemäß bevorzugt, bei dem Verbinden möglichst wenig eines niedrigmolekularen Klebers, vorzugsweise in einem Bereich von 0,01 bis 10 Gew.-%, vorzugsweise in einem Bereich von 0,01 bis 1 Gew.-% und besonders bevorzugt in einem Bereich von 0,01 bis 0,1 Gew.-%, jeweils bezogen auf das thermoplastische Polymer, oder gar keinen niedermolekularen Kleber, der bereits bei 30°C flüssig ist, einzusetzen. Damit beinhaltet der erfindungsgemäße Behälter in einer Ausgestaltung ein Verbundmaterial, das ein Lösungsmittel in einem Bereich von 0,005 bis 1 Gew.-%, vorzugsweise in einem Bereich von 0,005 bis 0,1 Gew.-% und besonders bevorzugt in einem Bereich von 0,005 bis 0,01 Gew.-%, jeweils bezogen auf das thermoplastische Polymer des Verbundmaterials, enthält. Weiterhin beinhaltet der erfindungsgemäße Behälter in einer anderen Ausgestaltung ein Verbundmaterial, das einen niedrigmolekularen Kleber in einem Bereich von 0,001 bis 1 Gew.-%, vorzugsweise in einem Bereich von 0,001 bis 0,1 Gew.-% und besonders bevorzugt in einem Bereich von 0,001 bis 0,01 Gew.-%, jeweils bezogen auf das thermoplastische Polymer des Verbundmaterials, enthält.

In einer bevorzugten Ausführungsform weist die Aluminiumfolie eine Maximaldicke oder einen Maximalquerschnitt zwischen 1 und 100 µm auf, bevorzugt zwischen 5 und 70 µm, besonders bevorzugt zwischen 10 und 30 µm. Ganz besonders bevorzugt weist die Aluminiumfolie eine Maximaldicke von 15 bis 25 µm auf.

Auch in den optional eingebrachten Prägungen weist die Aluminiumfolie eine Dicke in den angegebenen Bereichen auf. Durch die optionalen Prägungen kann lediglich der Verlauf der Aluminiumfolie mehrfach verändert werden, was zu einer Ausdehnung des Verlaufs der Aluminiumfolie bzw. des flächigen Verbundes führen kann. Die Aluminiumfolie kann durch die Prägungen beispielsweise einen wellenförmigen oder mäanderförmigen Verlauf annehmen. Wird der Querschnitt über diesen geprägten Bereich vermessen, der Wellenberge und Wellentäler enthält, so kann der Querschnitt deutlich von der ursprünglichen Aluminiumfolie abweichen. So kann die Aluminiumfolie nach einem Prägeprozess einen Bereich aufweisen, dessen Gesamtquerschnitt in einem Bereich zwischen 10 und 500 µm liegt, bevorzugt in einem Bereich zwischen 20 und 200 µm, besonders bevorzugt in einem Bereich zwischen 30 und 120 µm.

In einer erfindungsgemäßen Ausführungsform beinhaltet das Verbundmaterial eine weitere Polymerschicht. Die weitere Polymerschicht kann gleichfalls aus einem thermoplastischen Polymer bestehen. Die erste Polymerschicht und die weitere Polymerschicht können aus dem gleichen oder aus unterschiedlichen thermoplastischen Polymeren bestehen. Die Ausführungen zu thermoplastischen Polymeren im Zusammenhang mit der ersten Polymerschicht gelten auch für die weitere Polymerschicht und umgekehrt. Die weitere Polymerschicht kann sich beispielsweise auf der gegenüberliegenden Seite der Aluminiumfolie befinden, auf der die erste Polymerschicht aufgebracht ist. Alternativ kann sich die weitere Polymerschicht auf der gleichen Seite befinden wie die erste Polymerschicht. Zusätzlich zu der ersten und zweiten Polymerschicht können sich noch weitere Schichten, beispielsweise aus Aluminium, aus Wachs, aus einem Lack oder anderen Materialien auf der ersten oder der zweiten Seite der Aluminiumfolie befinden.

In einer Ausführungsform des erfindungsgemäßen Behälters ist die erste Polymerschicht oder die weitere Polymerschicht ein thermoplastisches Polymer, oft auch aus einem Harz, vorzugsweise einem Polyester, einem Wachs oder einer Mischung daraus. Unter Harz ist erfindungsgemäß eine Kohlenwasserstoffverbindung zu verstehen, die nicht in Wasser löslich ist. Als Harz werden unter anderem alle dem Fachmann unter dem Begriff synthetische Kunststoffe bekannte Substanzen gezählt. Harze sind beispielsweise Harnstoffharze, Alkydharze, Epoxidharze, Melaminharze, Phenolharze, Polyesterharze, Polyurethanharze, Polyamidharze und Vinylharze sowie mindestens zwei davon. Diese verschiedenen Harze weisen unterschiedliche Ausgangssubstanzen für die Herstellung der einzelnen Harze auf. Bei der Herstellung der synthetischen Kunststoffe reagieren die Ausgangssubstanzen mittels Kondensations- oder Additionsreaktion, zu den verschiedenen Harzen. Je nach Ausgangssubstanz und Reaktionsbedingungen können die Kunststoffe in einer Polymerisationsreaktion mit anschließender Aushärtung zu thermoplastischen oder duroplastischen Kunststoffen verarbeitet werden. Bevorzugt werden für die Polymerschicht Polyesterharze oder Alkydharze oder eine Mischung davon verwendet. Es können alternativ oder in Kombination mit den synthetischen Kunststoffen auch Naturharze eingesetzt werden.

Wachse sind erfindungsgemäß Kohlenwasserstoffverbindungen, die oberhalb 40 °C ohne Zersetzung schmelzen. Hierunter können sich auch Polyester befinden. Es werden pflanzliche, tierische und künstliche Wachse unterschieden. So zählen die Lipide zu pflanzlichen und tierischen Wachsen. Beispiele für künstliche Wachse sind Paraffin, Polyethylene, sowie dessen Copolymere.

In einer erfindungsgemäßen bevorzugten Ausführungsform ist das thermoplastische Polymer, vorzugsweise ein Polyester, bevorzugt ausgewählt aus der Gruppe bestehend aus einem Polycarbonat, einem Polyethylennaphthalat, einem Polybutylentherephthalat, einem Polyethylentherephthalat oder eine Mischung aus zwei oder mehreren davon.

In einer erfindungsgemäßen bevorzugten Ausführungsform ist das thermoplastische Polymer, vorzugsweise ein Polyester, bevorzugt ausgewählt aus der Gruppe bestehend aus einem Polycarbonat, einem Polyethylennaphthalat, einem Polybutylentherephthalat, einem Polyethylentherephthalat und einem Cellulosenitrat oder einer Mischung aus zwei oder mehreren davon.

Weiterhin ist es bevorzugt, dass die erste oder weitere Polymerschicht Molybdänsulfid (MoS₂) enthält. Bevorzugt weist die erste oder weitere Polymerschicht einen Gehalt an MoS₂ in einem Bereich von 1 bis 35 Gew.-%, besonders bevorzugt in einem Bereich von 2 bis 30 Gew.-%, ganz besonders bevorzugt in einem Bereich von 5 bis 25 Gew.-% auf. Es können ein oder mehrere weitere Substanzen, beispielsweise als Schmierstoff, dem thermoplastischen Polymer beigemischt werden. Die weiteren Substanzen können beispielsweise ausgewählt sein aus der Gruppe bestehend aus Graphit, Kupfer, Blei, Kunststoff und Keramik oder mindestens zwei davon.

Die Polymerschicht kann verschiedene Funktionen in dem Verfahren zur Herstellung eines Behälters und zur Bevorratung von analytischen Hilfsmitteln übernehmen. So kann die Polymerschicht, wenn sie vor dem Pressen der Aluminiumfolie vorgesehen wird, einen Einfluss auf das Verhalten der Aluminiumfolie während des Pressvorgangs ausüben. Wenn die Polymerschicht eher spröde ist, so kann sie dem weichen Aluminium in der Aluminiumfolie mehr Härte verleihen. Dies kann sich positiv auf das Dehnungsverhalten der Aluminiumfolie auswirken.

Die in diesem Zusammenhang bereitgestellte Aluminiumfolie weist vorzugsweise linienförmige Bereiche von Prägungen auf. Zum Einbringen der Prägungen kann beispielsweise ein Prägestempel mit einem Relief an der prägenden Oberfläche verwendet werden, um die verstärkten Bereiche zu erhalten. Dieses Relief kann beispielsweise eine wellenförmige Abfolge von Erhöhungen, oder Erhöhungen und Vertiefungen, aufweisen. Es können jedoch auch andere Formen von Reliefs verwendet werden, beispielsweise dreieckige, bevorzugt spitzwinklig dreieckige Reliefs, wobei der Spitze Winkel bevorzugt zur Oberfläche der Folie weist. Durch diese Erhöhungen und Vertiefungen in der Oberfläche des Prägestempels können Prägemuster in Oberflächen der Aluminiumfolie eingeprägt werden, solange das Material des Prägestempelreliefs härter ausgestaltet ist als die Oberfläche des zu prägenden Materials. Für solche Prägestempelreliefs wird beispielsweise eine Stahloberfläche verwendet. Wird der Prägestempel mit seinem Prägestempelrelief auf das weichere Material, wie Schaumstoff, gedruckt und dieses gegen eine glatte Unterlage gepresst, so überträgt sich das Muster des Prägereliefs von dem Prägestempel auf die Oberfläche des zu prägenden Materials. Es entsteht ein spiegelbildlichen Reliefmuster auf dem zu prägenden Material. Auf diese Weise kann beispielsweise ein Muster von verstärkten und geschwächten Bereichen in das zu prägende Material eingebracht werden. Wird dieses Muster von Vertiefungen und Erhöhungen auf die Aluminiumfolie eingebracht, so erreichen zunächst die Erhöhungen die Oberfläche der Aluminiumfolie und drücken Material, hier Aluminium, in eine andere Raumrichtung, sodass auf der anderen Seite eine Vertiefung, folglich ein Hohlraum, im Stempelrelief vorliegt.

In einer bevorzugten Ausführungsform ist der erfindungsgemäße Behälter erhältlich aus einer Aluminiumfolie mit Prägungen. Die Prägungen sind bevorzugt linienförmig in die Aluminiumfolie eingebracht und bilden vorzugsweise aus drei oder mehr linienförmigen Prägungen einen strahlenförmigen Ring. Hierbei gehen die linienförmigen Prägungen, auch als linienförmige Bereiche bezeichnet, meist von einem gedachten Mittelpunkt nach außen. Linienförmig bedeutet erfindungsgemäß, dass das eingeprägte Muster über einen länglichen Bereich, dessen Ausdehnung in eine Richtung um ein vielfaches größer ist als in eine zweite Richtung, eine Veränderung in die dritte Raumrichtung gegenüber der ursprünglichen Folie aufweist. Diese Vertiefung wird beispielsweise parallel oder auch schräg zu ihrer länglichen Ausdehnung durch Relieferhöhungen begrenzt. Dieses Muster kann sich parallel, quer oder schräg zu den Erhöhungen wiederholen, sodass mehrere linienförmige Bereiche erhalten werden. Je nach dem, ob die länglichen Relieferhöhungen parallel, schräg oder quer zueinander angeordnet sind, entstehen verschiedene Formen eines Reliefs mit linienförmigen Bereichen. Sind die Erhöhungen beispielsweise parallel zueinander angeordnet, so können kontinuierlich über das Material verlaufende linienförmige Bereiche erhalten werden. Sind die Erhöhungen beispielsweise schräg zueinander angeordnet, so kann ein Reliefmuster erhalten werden, dessen linienförmige Bereiche strahlenförmig in dem Material angeordnet sind.

In einer Ausführungsform ist der erfindungsgemäße Behälter erhältlich aus einer Aluminiumfolie, wobei die linienförmigen Bereiche strahlenförmig in der Aluminiumfolie vorgesehen sind. Sind die linienförmigen bzw. strahlenförmigen Bereiche dicht zueinander angeordnet, so wird eine kreisförmige Anordnung der linienförmigen Bereiche erhalten. In dieser Ausführungsform des Verfahrens, wird eine Aluminiumfolie bereitgestellt, wobei die linienförmigen Bereiche eine kreisförmige Fläche bilden. Weisen die linienförmigen Bereiche beispielsweise in dem Zentrum ihrer strahlenförmigen Anordnung einen nicht geprägten Bereich auf, so wird eine ringförmige Anordnung der linienförmigen Bereiche erhalten.

Der erfindungsgemäße Behälter ist erhältlich, indem die Aluminiumfolie, vorzugsweise mit mindestens einer Prägung, gepresst oder tiefgezogen wird.

Der erfindungsgemäße Behälter ist weiterhin erhältlich, indem die Aluminiumfolie, vorzugsweise mit mindestens einer Prägung, gepresst oder tiefgezogen wird, wobei ein Stempel vorzugsweise auf die mindestens eine Prägung wirkt. Bei dem Formen durch Pressen oder Tiefziehen wird eine Kontur- oder Profiländerung des zu verformenden Materials vorgenommen. Dies kann durch verschiedene Methoden erfolgen, die dem Fachmann zum Verformen von Aluminiumfolien bekannt sind. Das Formen ist ein Pressen oder Tiefziehen. Beispielsweise wird beim Pressen oder Tiefziehen ein Stempel auf das zu pressende Material in eine Pressform gedrückt. Je nach Ausgestaltung des Stempels und der Press-form kann ein unterschiedlich großer Spalt zwischen Stempel und Pressform gebildet sein. Dieser Spalt stellt den, dem zu verformenden Material zur Verfügung stehenden Platz bei dem Eindrücken des Stempels in die Pressform dar. Es hängt sowohl von der Ausgestaltung der Pressform und des Stempels als auch von der Gestalt, wie beispielsweise der Dicke, des zu verformenden Materials ab, welche Kräfte beim Verformungsprozess auf das zu verformende Material wirken. Beim Pressen können andere Kräfte wirken als beim Tiefziehen. So versteht man unter einem Pressvorgang das Eindrücken des zu verformenden Materials in eine vorgegebene Kontur, wobei sich die Dicke und Kontur des zu verformenden Materials ändern kann. Beim Tiefziehen, das auch als Zug/Druckumformen bezeichnet wird, wird die Dicke des zu verformenden Materials bevorzugt nicht verändert. Dies wird beispielsweise dadurch bewirkt, dass der Spalt bevorzugt breiter gewählt wird als die Dicke des zu verformenden Materials. Für weitere Details zum Formen von Materialien sowie der Vorgehensweise beim Formen wird an dieser Stelle auf folgende Literaturstellen verwiesen: "Umformtechnik, Band 3: Blechbearbeitung; Springer-Verlag, ISBN 3-540-50039-1 bzw. ISBN 0-387-50039-1" sowie " Handbuch der Fertigungstechnik; Carl Hanser Verlag 1986, ISBN 3-446-12536-1".

Die Form und das Material des Stempels einer Verformungsanlage können beliebig gewählt sein, solange eine ausreichende Härte zum Verformen durch Pressen oder Tiefziehen besteht. So kann der Stempel beispielsweise eine runde, eckige oder elliptische Form aufweisen. Der Stempel kann aus einem Material gefertigt sein, das nicht leicht verformbar ist, zumindest unter den Bedingungen des Pressens nicht in seiner Form verändert wird. Beispiele für Materialien aus dem der Stempel geformt sein kann sind Metalle oder Metallgemische oder Keramik, es können aber auch Kunststoffe eingesetzt werden, die ihre Form bei Erhöhung des Drucks auf den Stempel nicht ändern. Der Stempel kann massiv aber auch hohl ausgestaltet sein. Der Stempel besitzt eine Außenoberfläche, die hier auch als Profil bezeichnet wird, die mit dem zu pressenden Material während des Pressvorgangs wechselwirkt. Die Wechselwirkung findet beim Pressen weiterhin mit einer Pressform statt. Diese Pressform wechselwirkt mit dem zu pressenden Material auf der dem Stempel gegenüber liegenden Seite. Die Pressform weist eine Aushöhlung auf, in die der Stempel zusammen mit dem zu pressenden Material, meist die vorstehend näher beschriebenen Aluminiumfolie, gepresst wird. Die Pressform kann dabei eine dem Stempel als Gegenbild entsprechende Form aufweisen. Bevorzugt ist die Pressform als Ring ausgestaltet. Sie kann aber auch zumindest an einigen Stellen eine von dem Stempelprofil abweichende Gegenform darstellen.

Bevorzugt weist die Pressform eine durchgehende Kante auf, in die das zu pressende Material mit Hilfe des Stempels eingedrückt wird. Beispiele für die Form der Pressform sind kreisförmig, oval, wellenförmig, sternförmig oder eckig. Besonders bevorzugt bildet die Pressform einen Kreis.

Der erfindungsgemäße Behälter ist vorzugsweise erhältlich, in dem der Stempel zusammen mit der Aluminiumfolie in die Pressform gepresst wird, sodass eine Vertiefung in der Aluminiumfolie entsteht. Beispielsweise kann hierfür eine Pressvorrichtung angewendet werden, die einen Stempel und eine Pressform vorsieht, die so in ihrer Form aneinander angepasst sind, dass das Material das sich bei dem Pressvorgang zwischen Stempel und Pressform befindet mindestens, in einer Ausdehnungsrichtung verformt wird. Beispielsweise kann die Aluminiumfolie, die sich in ihrer Länge und Breite jeweils in eine Richtung ausdehnt, durch den Pressvorgang in eine dritte Richtung, quer zu ihrem ursprünglichen Verlauf gepresst werden. Dabei kann die Folie in der Querrichtung um verschiedene Beträge abgelenkt werden. Beispielsweise kann es sich bei der Ablenkung der Folie um ein oder mehrere cm oder ein oder mehrere mm handeln. Je nachdem wie der Stempel ausgestaltet ist und wie er in die Form gepresst wird, kann die Folie an den gepressten bzw. tiefgezogenen Bereichen dünner ausgestaltet sein als vor dem Pressvorgang. Um ein Reißen der Folie beim Pressen oder Tiefziehen zu vermindern, können Prägungen in den zu formenden Bereichen der Folie eingebracht sein. Diese Prägungen erleichtern einen Materialfluss der Folie während des Press- bzw. Tiefziehprozesses.

Bevorzugterweise passen Stempel und Pressform so genau in einander, dass nur wenig Abstand zwischen ihnen für das zu pressende Material bleibt. Dabei ist der Durchmesser der Pressform um wenige µm größer ist als der Durchmesser des Stempels. Der Abstand zwischen dem Stempel und der Passform wird auch als Spaltmaß bezeichnet. Beispielsweise kann Spaltmaß nur wenige µm betragen, beispielsweise zwischen 0,1 und 100 µm, bevorzugt zwischen 1 und 50 µm, besonders bevorzugt zwischen 15 und 40 µm. Durch das Pressen der Aluminiumfolie mit Hilfe des Stempels in die Passform, wird vorzugsweise eine Vertiefung in die Aluminiumfolie gepresst. Je nach Materialzusammensetzung und Prägemuster kann die Vertiefung unterschiedlich tief ausgestaltet werden. Die so entstandene Vertiefung hat vorzugsweise eine zylindrische Form. Sie kann jedoch auch alle anderen durch die Geometrie des Stempels und der Passform festgelegte Formen annehmen.

In einer erfindungsgemäßen Ausführungsform hat die Vertiefung eine Tiefe in einem Bereich von 1 bis 10 mm, bevorzugt in einem Bereich von 2 bis 7 mm und besonders bevorzugt in einem Bereich von 2 bis 5 mm. Die eingeprägten linienförmigen Bereiche in der Aluminiumfolie erleichtern den Pressvorgang zu, im Vergleich zur Dicke der Aluminiumfolie, recht großen Vertiefung. Durch die geprägten linienförmigen Bereiche wird erreicht, dass beim Pressvorgang die Aluminiumfolie entlang der Prägungen besser in die Pressform gleiten kann. Darüber hinaus kann sich Material aus dem geprägten Bereich beim Formen, insbesondere beim Pressen oder Tieferziehen, über den zu verformenden Bereich verteilen. Nach dem Formen können die Prägungen minimiert oder glattgezogen oder glattgepresst sein. Der Gleitprozess kann durch weitere Hilfsmittel, wie Aufbringen einer Wachsschicht auf die Aluminiumfolie erleichtert werden. Auf diese Weise wird verhindert, dass die Aluminiumfolie aufgrund des Pressdrucks reißt. Insgesamt kann so ein höherer Pressdruck auf die Aluminiumfolie ausgeübt werden, also eine tiefere Vertiefung in der Aluminiumfolie erreicht werden als in nicht geprägte Aluminiumfolien. Dies wird unter anderem dadurch erreicht, dass während des Press- bzw. Tiefziehprozesses eine Materialumverteilung innerhalb der geprägten Bereiche stattfindet, sodass anschließend eine nahezu glatte Folie erhalten wird. Die Falten in dem geprägten Bereich werden bei diesem Prozess geglättet.

Durch die Vertiefung wird eine Geometrie der Aluminiumfolie geschaffen, die es ermöglicht auch dreidimensionale Gegenstände damit formschlüssig zu umgeben. So können auf diese Weise Fassungen mit analytischen Hilfsmitteln umgeben werden, um bevorzugt einen sterildichten Behälter zu erhalten.

Unter analytischen Hilfsmitteln sind allgemein im Rahmen der vorliegenden Erfindung Hilfsmittel zu verstehen, welche bei in WO 2010/094426 beschriebenen analytischen Funktionen unterstützend eingesetzt werden können. Insbesondere kann es sich bei den analytischen Hilfsmitteln um medizinische und/oder diagnostische Hilfsmittel handeln, insbesondere um Hilfsmittel, welche eingerichtet sind, um bei einem qualitativen und/oder quantitativen Nachweis mindestens eines Analyten in einer Köperflüssigkeit eines Probanden, wie beispielsweise eines oder mehrerer der folgenden Analyten: Glucose, Lactat, Triglyceride, Gerinnungsparameter und Cholesterin, eingesetzt zu werden. Bei der Köperflüssigkeit eines Probanden kann es sich beispielsweise um Blut, interstitielle Flüssigkeit, Urin oder ähnlichen Körperflüssigkeiten handeln. Insbesondere können die analytischen Hilfsmittel als Einweg-Hilfsmittel (Disposables) ausgestaltet sein, also für den einmaligen Gebrauch bestimmt sein. Die analytischen Hilfsmittel können dementsprechend beispielsweise mindestens ein Stechelement, beispielsweise in Form einer Lanzette umfassen, also ein Element, welches eingerichtet ist, um mindestens eine Öffnung in einer Haut des Probanden zu erzeugen. Diese Öffnung in der Haut des Probanden kann beispielsweise in einem Ohrläppchen, einer Fingerbeere oder einem Unterarm des Probanden erfolgen. Beispielsweise können diese Lanzetten ein oder mehrere Nadelspitzen und/oder angeschliffenen Spitzen umfassen. Auch andere scharfkantige Elemente können alternativ oder zusätzlich verwendet werden, beispielsweise Klingen, scharfkantige Spitzen oder ähnliches. Die Lanzetten können beispielsweise aus stabförmigen Ausgangsmaterialien hergestellt werden, beispielsweise in Form von nadelförmigen Lanzetten. Besonders bevorzugt im Rahmen der vorliegenden Erfindung ist jedoch die Verwendung einer oder mehrerer Lanzetten, welche aus plattenförmigen Materialien, insbesondere Metallblechen, hergestellt sind.

Alternativ oder zusätzlich zu Lanzetten können die analytischen Hilfsmittel auch jeweils ein oder mehrere Testfelder umfassen. Diese Testfelder weisen mindestens eine Testchemie auf, welche eingerichtet ist, um bei Anwesenheit mindestens eines nachzuweisenden Analyten mindestens eine messbare Eigenschaft zu ändern. Diese Testchemie, welche eingerichtet ist, um alleine oder in Zusammenwirkung mit dem Analyten und/oder weiteren Hilfsstoffen die Anwesenheit oder - was hiervon umfasst sein soll - die Abwesenheit des mindestens einen Analyten zu indizieren, kann auf verschiedene Weisen ausgestaltet sein. Diesbezüglich kann beispielsweise auf WO 2007/012494 A1 verwiesen werden, in welcher besonders feuchtigkeitsstabile Testchemien beschrieben werden. Die in dieser Druckschrift genannten Testchemien können, einzeln oder in Kombination, auch im Rahmen der vorliegenden Erfindung eingesetzt werden. Insbesondere können stark spezifische Testchemien eingesetzt werden, bei welchen der Nachweis spezifisch auf den mindestens einen Analyten reagiert. Die mindestens eine messbare Eigenschaft, aus deren Messung sich qualitativ oder quantitativ der mindestens eine Analyt nachweisen lässt, kann beispielsweise mindestens eine elektrochemische Eigenschaft und/oder mindestens eine optische Eigenschaft umfassen.

Weiterhin lassen sich die analytischen Hilfsmittel auch derart ausgestalten, dass diese als kombinierte Testelemente ausgestaltet sind. So können beispielsweise kombinierte Testelemente mit mindestens einer Lanzette und mindestens einem Testfeld, beinhaltend mindestens eine Testchemie, verwendet werden, wobei die Testchemie eingerichtet sein kann, um bei Anwesenheit des mindestens einen nachzuweisenden Analyten mindestens eine messbare Eigenschaft zu ändern. Beispielsweise kann das Testelement unmittelbar in die Lanzette integriert sein. So kann beispielsweise die Testchemie am Ende der Lanzette aufgenommen sein und/oder Teile der Lanzette bedecken. In einer bevorzugten erfindungsgemäßen Ausführungsform weist das analytische Hilfsmittel ein Stechelement, beispielsweise in Form einer Lanzette, oder ein Testfeld zum Nachweis eines Analyten in der Körperflüssigkeit oder beides auf.

Das mindestens eine analytische Hilfsmittel ist dabei vorzugsweise in einem analytischen Magazin angeordnet. Analytische Magazine können unterschiedlichste Formen aufweisen. So sind vor allem Magazine in Stapel-, Disc-, oder Bandform bekannt Das analytische Magazin ist beispielsweise eingerichtet, um eine Mehrzahl von analytischen Hilfsmitteln in einer Mehrzahl von Ausnehmungen aufzunehmen. Unter einem analytischen Magazin ist somit vorzugsweise eine Vorrichtung zu verstehen, welche als Einheit gehandhabt werden kann, welche beispielsweise ein gemeinsames Gehäuse aufweisen kann und welche allgemein in der Medizintechnik einsetzbar sein kann. Unter analytisch ist dabei allgemein die Möglichkeit eines Einsatzes für den qualitativen und/oder quantitativen Nachweis mindestens eines Analyten und/oder die Bestimmung mindestens einer weiteren messbaren Eigenschaft zu verstehen. Insbesondere kann unter analytisch somit eine diagnostische Eigenschaft verstanden werden, also eine Verwendung für die Bestimmung mindestens einer Eigenschaft eines Körpers und/oder eines Bestandteils eines Körpers eines Probanden. Das analytische Magazin kann entsprechend in einem analytischen System eingesetzt werden. Beispielsweise kann es sich bei einem derartigen System um ein Messgerät handeln, mittels dessen mindestens ein Analyt, beispielsweise mindestens ein Metabolit, in einer Körperflüssigkeit des Probanden qualitativ und/oder quantitativ nachgewiesen wird. Beispielsweise kann es sich bei diesen Systemen um Blutglukosemessgeräte handeln, wie sie hinlänglich im Handel bekannt sind, wie beispielsweise AccuChek^{®} Mobile, AccuChek^{®} Active oder AccuChek^{®} Go.

Alternativ oder zusätzlich ist es jedoch auch möglich, dass die Lanzette und das Testfeld getrennt ausgebildet sind, beispielsweise jeweils mindestens eine Lanzette und getrennt davon jeweils mindestens ein Testfeld pro Ausnehmung des analytischen Magazins. Diese Teile des analytischen Hilfsmittels können beispielsweise auch getrennt handhabbar sein, so dass beispielsweise die Lanzette von einem Aktor eines Systems gehandhabt werden kann, um eine Stechbewegung und/oder eine Sammelbewegung auszuführen, während das Testfeld bzw. das Testelement beispielsweise unverändert verbleibt, beispielsweise innerhalb der Ausnehmung. So kann beispielsweise das System eingerichtet sein, um eine Stech- und/oder Sammelbewegung mittels der mindestens einen Lanzette und/oder einem in der Lanzette optional enthaltenen Kapillarelement durchzuführen, so dass während eines Stechvorgangs und/oder während einer Probenahmebewegung direkt Körperflüssigkeit von der Lanzette aufgenommen werden kann. Dabei kann zunächst ein Einstich in die Haut des Probanden erzeugt werden, Körperflüssigkeit gesammelt und diese dann, beispielsweise bei einer Rückwärtsbewegung der Lanzette, zurück in die Ausnehmung hinein, auf das Testfeld übertragen werden. Auch andere Ausgestaltungen sind möglich. In einer erfindungsgemäßen Ausführungsform weist das analytische Hilfsmittel ein Testfeld und ein Stechelement auf, die miteinander in Kontakt stehen, um Flüssigkeit miteinander auszutauschen.

Alternativ oder zusätzlich zu Lanzetten und/oder Testfeldern können die analytischen Hilfsmittel weitere Elemente umfassen, welche einem Analysezweck dienen. So können beispielsweise Transferelemente und/oder Sammelelemente umfasst sein, welche dem Zweck einer Aufnahme und/oder eines Transports von Körperflüssigkeit dienen. Beispielsweise kann mittels derartiger Transportelemente und/oder Sammelelemente eine Aufnahme von Blut und/oder interstitieller Flüssigkeit von einer Haut des Probanden und/oder einer Stelle innerhalb des Körpers des Probanden und/oder einer Stelle auf der Haut des Probanden erfolgen und/oder ein Transport zu einem Testelement, insbesondere einem oder mehreren Testfeldern. Ein derartiger Transport kann beispielsweise durch eine Transportbewegung erfolgen, mittels eines oder mehrerer Transportelemente, welche beweglich ausgestaltet sind und eine Menge der Probe der Körperflüssigkeit aufnehmen und transferieren können. Alternativ oder zusätzlich können auch andere Transportelemente und/oder Sammelelemente vorgesehen sein, beispielsweise Kapillaren und/oder Elemente mit Kapillarwirkung. Beispielsweise kann es sich dabei um geschlossene Kapillaren oder um Kapillarkanäle, insbesondere Kapillarspalte, handeln. Kombinierte analytische Hilfsmittel, welche mindestens eine Lanzettenfunktion und mindestens eine Kapillarfunktion aufweisen, werden im Folgenden auch als Microsampler bezeichnet.

In einer weiteren erfindungsgemäßen Ausführungsform weist das analytische Hilfsmittel eine hydrophile Beschichtung auf. Dies kann beispielsweise der zuvor erwähnte Kapillarkanal zwischen Lanzette und Testfeld sein. Alternativ kann auch die Lanzette oder das Testfeld mindestens einen Bereich aufweisen, der hydrophil beschichtet wurde. Hierdurch wird ein verbesserter Transport der Körperflüssigkeit auf dem analytischen Hilfsmittel ermöglicht.

In einer Ausführungsform des Behälters, ist eine Vielzahl analytischer Hilfsmittel in einer ringförmigen Fassung vorgesehen. Häufig stellt diese eine Magazinierung der analytischen Hilfsmittel dar. Im Speziellen handelt es sich hierbei um eine kreisförmige Ausgestaltung der analytischen Hilfsmittel. Dies hat zu Folge, dass bevorzugterweise auch das Magazin eine kreisförmige Ausgestaltung in Form einer Disk annimmt.

Es ist erfindungsgemäß besonders bevorzugt, wenn die analytischen Hilfsmittel derart in den Ausnehmungen aufgenommen sind, dass in einer Ausnehmung genau ein analytisches Hilfsmittel aufgenommen ist. Umfasst das analytische Hilfsmittel selbst jeweils eine Mehrzahl analytischer Teil-Hilfsmittel, wie beispielsweise jeweils mindestens eine Lanzette und jeweils mindestens ein Testfeld, so können beispielsweise das jeweils mindestens eine Testfeld und/oder die jeweils mindestens eine Lanzette, welche für einen einzigen, gemeinsamen Test vorgesehen sind (beispielsweise eine einzige Aufnahme von Körperflüssigkeit und/oder Analyse von Körperflüssigkeit) in einer gemeinsamen Ausnehmung aufgenommen sein. Diese Ausgestaltung, bei welcher in einer Ausnehmung jeweils ein analytisches Hilfsmittel aufgenommen ist, beispielsweise mit jeweils mindestens einem Teil-Hilfsmittel in Form eines Testfelds und/oder mit jeweils mindestens einem Teil-Hilfsmittel in Form einer Lanzette, ist insbesondere bei einem scheibenförmigen Magazin realisierbar oder auch bei anderen Ausgestaltungen von Magazinen, wie beispielsweise stangenförmigen Magazinen. In einer bevorzugten Ausführungsform sind jeweils eine Lanzette und ein Testfeld so zueinander angeordnet, dass eine Flüssigkeit, beispielsweise in Form von Blut, die an der Lanzette anhaftet auf das Testfeld übertragbar ist. Dies kann, dadurch bewerkstelligt werden, dass die Lanzette und das Testfeld übereinander angeordnet sind, sodass durch Ausüben eines leichten Drucks auf Lanzette oder das Testfeld die Flüssigkeit, in diesem Beispiel das Blut, auf das Testfeld direkt übertragen werden kann.

Alternativ zu einer Ausgestaltung, bei welcher jedes analytische Hilfsmittel in einer separaten Ausnehmung aufgenommen ist, ist auch eine Ausgestaltung möglich, bei welcher mehrere analytische Hilfsmittel gleicher Art oder verschiedener Art in einer Ausnehmung aufgenommen sind. Ein Beispiel einer derartigen Ausgestaltung ist ein Bandmagazin, bei welchem ein Gutwickel mit einer Mehrzahl unbenutzter analytischer Hilfsmittel in einer ersten Ausnehmung aufgenommen ist und ein Schlechtwickel mit einer Mehrzahl benutzter analytischer Hilfsmittel in einer zweiten Ausnehmung. Auch andere Ausgestaltungen sind möglich.

Unter einer Ausnehmung ist dabei allgemein ein Element zu verstehen, welches mindestens einen zumindest teilweise geschlossenen Hohlraum aufweist, in welchem das analytische Hilfsmittel aufgenommen sein kann. Die Ausnehmung kann auch als Kammer bezeichnet werden. Der Hohlraum kann dabei auch eine oder mehrere Öffnungen umfassen. Die Ausnehmungen können auch jeweils eine oder mehrere Teil-Ausnehmungen umfassen und können jeweils eine oder mehrere Ausnehmungswände umfassen, welche einem Innenraum der Ausnehmungen zuweisen.

Nachdem das analytische Hilfsmittel in die Ausnehmung der Fassung eingebracht ist und die Öffnungen mit der Aluminiumfolie abgedeckt wurde, wird der Behälter verschlossen. Unter verschlossen wird vorzugsweise ein hermetisches Verschließen verstanden, um die analytischen Hilfsmittel steril halten zu können. Der Behälter wird anschließend, bevorzugterweise in einem Heißsiegelverfahren, hermetisch abgedichtet. Es können jedoch auch andere Verfahren, wie Laser-Schweißverfahren oder Klebeverfahren, verwendet werden.

Die Polymerschicht kann dazu beitragen, dass die Hydrophilie des analytischen Hilfsmittels während seiner Bevorratung innerhalb des Behälters wenn überhaupt nur unwesentlich leidet. Dies kann besonders wichtig bei zu bevorratenden analytischen Hilfsmitteln sein, die eine hydrophile Beschichtung aufweisen. Durch die Wahl der Materialien, die für den Behälter getroffen wird, kann das analytische Hilfsmittel möglichst lange in seinem Originalzustand aufbewahrt werden kann. In einer weiteren erfindungsgemäßen Ausführungsform wird der Behälter so ausgestaltet, dass das analytische Hilfsmittel vor Benutzung hydrophil gehalten wird. Dies kann beispielsweise erreicht werden, indem auf beide Seiten der Aluminiumfolie eine Polymerschicht aufgebracht wird. Bevorzugterweise handelt es sich hierbei um eine Polyesterschicht.

In einer weiteren erfindungsgemäßen Ausführungsform weist die erste Polymerschicht oder die weitere Polymerschicht oder jeweils beide eine Dicke in einem Bereich von 0,5 bis 20 µm, bevorzugt in einem Bereich von 0,5 bis 10 µm, weiterhin bevorzugt in einem Bereich von 2 bis 8 µm und besonders bevorzugt in einem Bereich von 3 bis 6 µm auf.

In einem weiteren Aspekt der Erfindung wird ein Verfahren zur Herstellung eines Behälters zur Bevorratung von analytischen Hilfsmitteln vorgeschlagen, beinhaltend die Schritte:
- Bereitstellen einer Fassung mit einer Vielzahl analytischer Hilfsmittel,
- Abdecken mindestens eines Teils der Fassung mit einem flächigen Verbundmaterial beinhaltend eine Aluminiumfolie die mit einer Polymerschicht verbunden ist, wobei die Polymerschicht zu der Fassung weist,
- Erhitzen mindestens der Polymerschicht sodass die Polymerschicht mindestens teilweise aufschmilzt und mit der Fassung eine Verbindung eingeht und einen Behälter bildet, wobei vor oder beim Abdecken mindestens eines Teils der Fassung mit dem flächigen Verbundmaterial, die Aluminiumfolie gepresst oder tiefgezogen wird.

Die Aluminiumfolie wird bei einem Formen gepresst oder tiefgezogen. Diese Art der Verformung findet bevorzugt vor dem Abdecken eines Teils der Fassung statt. Es ist jedoch auch denkbar die Fassung aus einem Material zu fertigen, dass sie dazu geeignet macht, in einem Press- oder Tiefziehvorgang der Aluminiumfolie als Stempel genutzt zu werden. Wie bereits erwähnt, kann die Polymerschicht vor oder nach dem Verformungsprozess mit der Aluminiumfolie verbunden werden.

Weiterhin kann in einem weiteren Verfahrensschritt mindestens eine weitere Polymerschicht mit der Aluminiumfolie direkt oder indirekt verbunden werden. Unter direkt wird ein unmittelbarer Kontakt der weiteren Polymerschicht mit der Aluminiumfolie verstanden, während bei dem indirekten Verbinden kein unmittelbarer Kontakt zwischen Aluminiumfolie und weiterer Polymerschicht besteht.

In einer weiteren Ausführung des Verfahrens besteht die Polymerschicht aus einem thermoplastischen Polymer. Bevorzugt ist das thermoplastische Polymer ein Polyester.

In einer weiteren Ausführung des Verfahrens ist das Polyester ausgewählt aus der Gruppe bestehend aus: Polycarbonat, Polyethylennaphthalat, Polybutylentherephthalat, Polyethylentherephthalat und Polyesterharz oder einer Mischung aus mindestens zwei davon.

In einer weiteren Ausführung des Verfahrens ist das Polyester ausgewählt aus der Gruppe bestehend aus: Polycarbonat, Polyethylennaphthalat, Polybutylentherephthalat, Polyethylentherephthalat, Cellulosenitrat und Polyesterharz oder einer Mischung aus mindestens zwei davon.

In einem Beispiel wird ein Verfahren zur Herstellung eines Behälters, vorzugsweise mindestens teilweise aus einem Aluminium beinhaltenden Verbund vorgeschlagen, wobei das Verfahren die Schritte beinhaltet:
- Bereitstellen einer Aluminiumfolie,
- Pressen der Aluminiumfolie, sodass eine Vertiefung in der Aluminiumfolie entsteht,
- Einbringen eines analytischen Hilfsmittels in die Vertiefung,
- Verschließen des Behälters, vorzugsweise zum Ausbilden einer Verpackung für das analytische Hilfsmittel, die dieses von der Umgebung abtrennt.

Von Abtrennen ist bevorzugt die Rede, wenn die Verpackung das analytische Hilfsmittel von allen Seiten umgibt und somit ganz von der Umgebung abtrennt, um so beispielsweise eine sterile und oft auch gasdichte Verpackung zu gewährleisten.

Für die Ausgestaltung der Aluminiumfolie wird auf die Ausführungen zu dem erfindungsgemäßen Behälter verwiesen.

Das Pressen der Aluminiumfolie wird auch als Tiefziehen bezeichnet und erfolgt vorzugsweise wie zuvor beschrieben.

Im Zusammenhang mit dieser Verfahrensausgestaltung ist es bevorzugt, dass als Vertiefung ein Raum bezeichnet wird, der durch mindestens zwei zueinander abgewinkelte Flächen der gepressten Aluminiumfolie bzw. des gepressten Verbundmaterials aufgespannt wird. Die zueinander abgewinkelten Flächen entstehen bevorzugt durch das Abknicken der Aluminiumfolie an der Innenseite des Knicks bei dem Pressvorgang. Die Vertiefung kann dabei eine Ausgestaltung der Aluminiumfolie bzw. des Verbundmaterials sein, die durch mindestens zwei bevorzugt zu einander abgewinkelte Oberflächen der Aluminiumfolie oder des Verbundmaterials ausgebildet wird und in die weiteren Raumrichtungen offen sein kann.

Die nach dem Pressen zu einander abgewinkelten Oberflächen können dabei Abmessungen in einem Bereich von 1 mm bis 0,5 m, bevorzugt in einem Bereich von 1 cm bis 30 cm, besonders bevorzugt in einem Bereich von 2 cm bis 20 cm senkrecht zum Knick, der durch das Pressen entsteht, aufweisen.

In diese Vertiefung wird erfindungsgemäß das analytische Hilfsmittel eingebracht. Dies kann in Form von einzelnen analytischen Hilfsmitteln geschehen, wie sie zuvor beschrieben wurden, oder in Form einer Vielzahl von analytischen Hilfsmitteln. Eine Vielzahl analytischer Hilfsmittel kann beispielsweise durch eine Fassung, wie zuvor erwähnt und definiert, zusammengehalten werden.

Das Verschließen des Behälters kann vorzugsweise durch Verkleben der Aluminiumfolie, wie zuvor beschrieben oder durch Aufschmelzen einer Polymerschicht, die mit der Aluminiumfolie verbunden ist, wie zuvor beschrieben, durch Erhitzen vorgenommen werden. Bevorzugt wird die Polymerschicht durch Aufschmelzen mit der Fassung verschmolzen.

Zudem gelten die Ausführungen zu dem erfindungsgemäßen Behälter ebenso entsprechend für das erfindungsgemäße Verfahren zur Herstellung eines Behälters. Dies gilt insbesondere für Materialien und räumliche Ausgestaltungen.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1a:: Schematische Darstellung einer Anordnung eine Aluminiumfolie und einer Polymerfolie;
- Figur 1b:: Schematische Anordnung einer geprägten Aluminiumfolie zwischen einem Stempel und einer Pressform;
- Figur 2a:: Photo eines Prägewerkzeugs mit gefrästem Unterteil, Deckel und Schaumstoff;
- Figur 2b:: Photo einer Tiefpresse mit Stempel und Pressform;
- Figur 2c:: Photo einer unbehandelten Aluminiumfolie, einer geprägten Aluminiumfolie und eines tiefgepressten Aluminiumfoliebehälters;
- Figur 2d:: Schematische Darstellung einer Fassung mit analytischen Hilfsmitteln befüllt, die mit einer tiefgepressten Folie bedeckt wird;
- Figur 2e:: Photo einer Fassung, die mit zwei gepressten Folien versiegelt ist; die zusammen mit der Fassung einen Behälter für analytische Hilfsmittel bilden;
- Figur 3:: Schematische Darstellung eines Presswerkzeugs mit einem Stempel, der sich in der Pressform befindet und beide zusammen ein Spaltmaß bilden;
- Figur 4a: Schematische Darstellung eines eckigen Prägemusters in einer Aluminiumfolie;
- Figur 4b: Schematische Darstellung eines wellenförmigen Prägemusters in einer Aluminiumfolie;
- Figur 5: Photo eines Deckels eine Druckluftpresse mit eingraviertem Prägemuster;
- Figur 6: Photo eines Druckraums einer Druckluftpresse mit Druckluftanschluss und Gummidichtung;

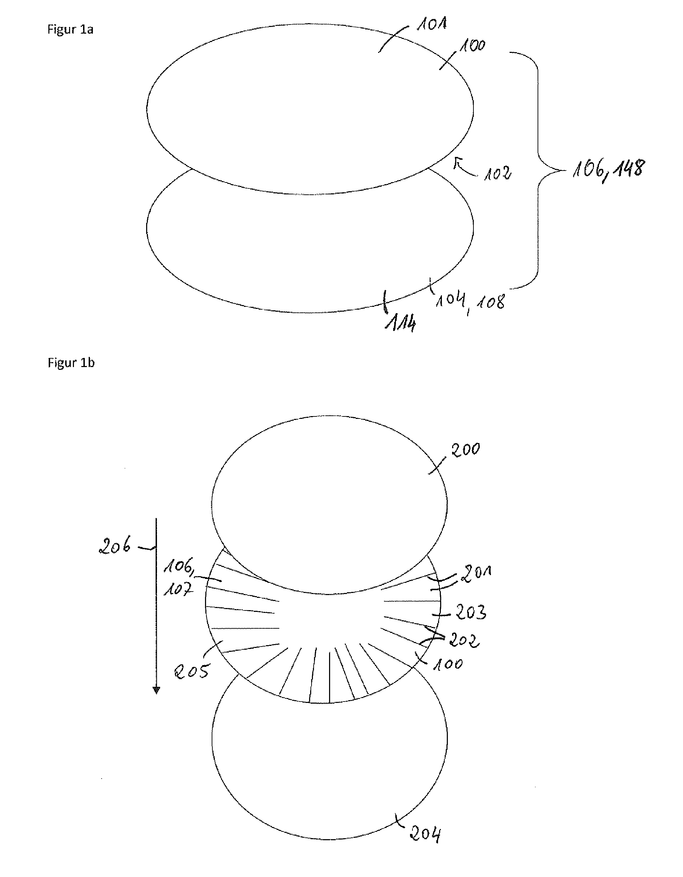

In Figur 1a ist schematisch eine Aluminiumfolie 100 gezeigt, die eine runde Form aufweist und einen Teil für den erfindungsgemäßen Behälter 146 darstellt. Diese Aluminiumfolie 100 kann auf mindestens einer der beiden Seiten 101 und 102 mit einer ersten Polymerschicht 104, beispielsweise mit einer Polymerfolie 104 verbunden werden. In der Figur 1a ist nur die Anordnung einer Polymerfolie 104 auf der zweiten Seite 102 gezeigt. Es ist jedoch auch möglich die Polymerfolie 104 oder eine weitere Polymerschicht 114 alternativ oder zusätzlich auf die erste Seite 101 aufzubringen. Hieraus ergibt sich ein Aluminium/Polymerverbund 106, der im Folgenden meist Verbund 106 oder nur Aluminiumfolie 106 genannt wird. Es können sich darüberhinaus weitere Schichten zwischen, ober- oder unterhalb der gezeigten Folien 100, 104 befinden. So ist bei einer später noch zu erwähnenden Aluminiumfolie 100, 106 ohne Prägungen 202 weiterhin mindestens eine Wachsschicht 108 auf die Aluminiumfolie 100 bzw. den Verbund 106 aufgebracht. Diese Wachsschicht 108 befindet sich vorzugsweise auf der gegenüberliegenden Seite zu der Polymerschicht 104, wenn es sich um einen Verbund 106 mit nur einer Polymerfolie 104 handelt oder auf der Fassung 113 abgewandten Seite, wenn beide Seiten der Aluminiumfolie 100, 106 mit Polymerfolie 104 bedeckt sind. Es können sich aber auch auf beiden Seiten 101 und 102 der Aluminiumfolie 100 jeweils eine Wachsschicht 108 befinden. Über oder unter der optionalen Wachsschicht 108 kann sich eine weitere Schicht eines Schutzlackes 114 befinden. In einem Beispiel wurde als Schutzlack 114 ein Cellulosenitrat enthaltender Lack verwendet. Hierzu wurde eine alkoholische Lösung mit 20 Gew.-% Cellulosenitrat (bekannt als Zaponlack der Firma Carl Roth) und 10 bis 25 Gew.-% MoS₂ bereitet. Dieser Schutzlack 114 wurde in einer solchen Menge aufgetragen, dass nach Trocknung des Schutzlackes 114 eine Schicht mit einer Dicke von 5 µm resultierte. Der Schutzlack 114 kann gegen Korrosion der Aluminiumfolie einerseits und gegen mechanische Belastung beispielsweise bei der Herstellung oder Anwendung der Aluminiumfolie schützen. Die Verbindung der Aluminiumfolie 100 mit der Polymerschicht 104 kann wie bereits erwähnt vor einem optionalen Prägeschritt erfolgen oder im Anschluss daran. Durch das Verbinden der Aluminiumfolie 100 mit der Polymerschicht 104 wird ein Aluminium/Polymerverbund 106 in Form eines flächigen Verbundmaterials 148 geschaffen. Eine solche Aluminiumfolie 100 oder Aluminium/Polymerverbund 106 ist dazu geeignet in einem Rolle-zu-Rolle Verfahren verarbeitet zu werden. Dies ist hier jedoch nicht gezeigt.

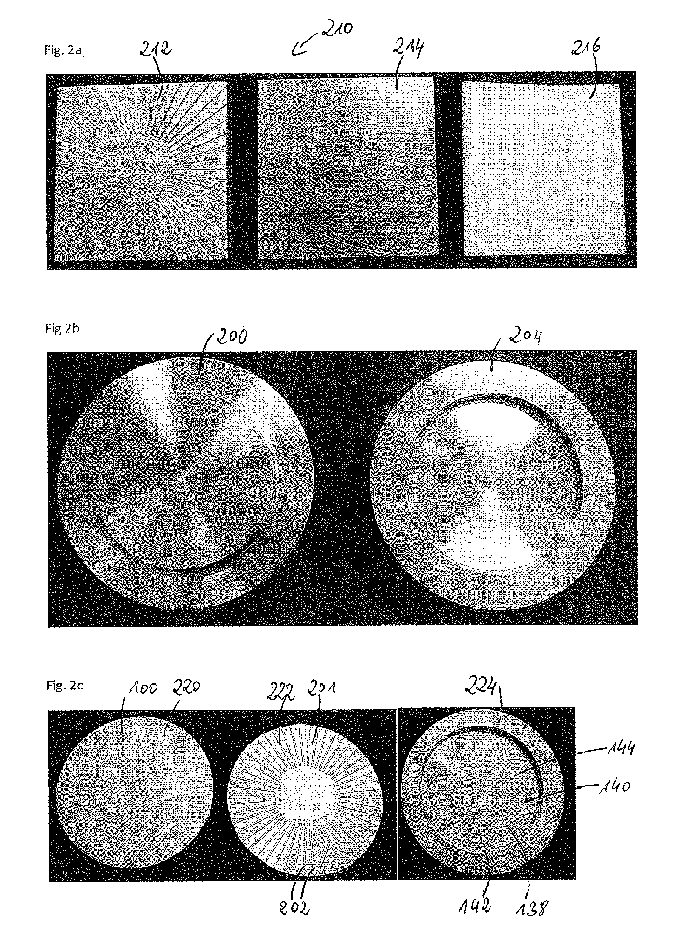

Die Aluminiumfolie 100 oder der Aluminium/Polymerverbund 106 wird mit Hilfe eines Prägewerkzeugs 210 aus Figur 2a ein Prägemuster 201 eingeprägt. Dieses Prägemuster 201 besteht aus mehreren Prägungen 202, beispielswies in Form von linienförmig geprägten Bereichen 202. In diesem speziellen Fall sind die linienförmigen Prägungen 202 strahlenförmig im Kreis angeordnet und bilden eine kreisförmige Fläche 203 wie in Figur 1b gezeigt. Für das Prägen kann, wie bereits erwähnt, das Prägewerkzeug 210 aus Figur 2a verwendet werden. Beim Prägen wird ein gefrästes Bauteil 212 mit einem spiegelbildlichen Prägemuster 201 auf die Folie 100, 106 gepresst, wobei der Deckel 214 auf der anderen Seite der Folie 100, 106 gegengehalten wird. Es kann ein Schaumstoff 216 zwischen Deckel 214 und gefrästem Bauteil 212 gelegt werden, um die Folie 100, 106 in Form zu pressen.

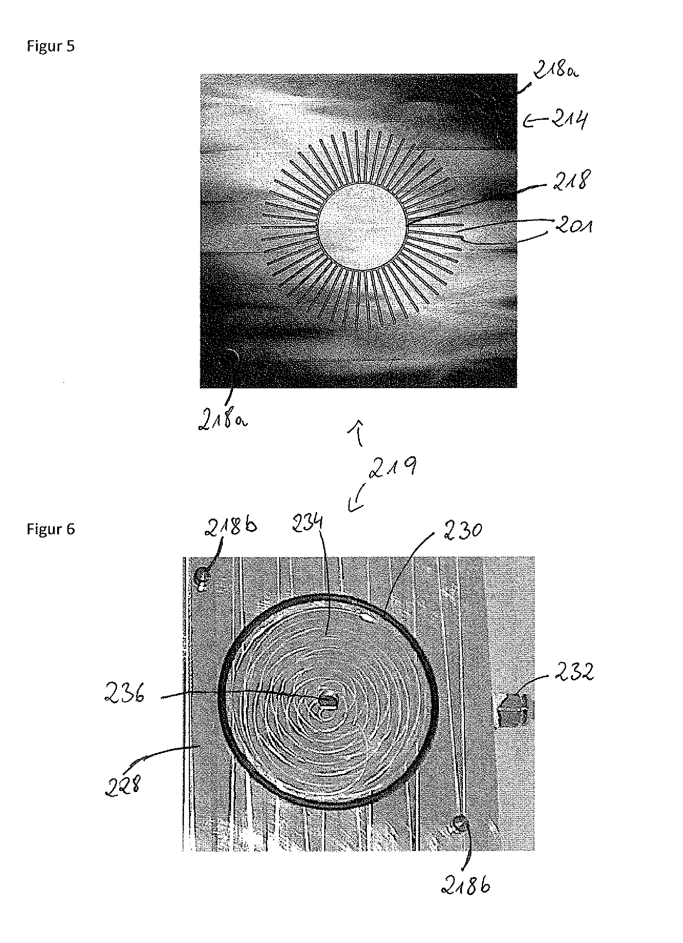

Ein alternativer Weg Prägungen 202 in die Aluminiumfolie 100, 106 einbringen zu können ist mit den Werkzeugen aus Figuren 5 und 6 möglich. Hier ist in Figur 5 ein Deckel 214 einer Druckluftpresse 219 gezeigt, die in dem Deckel 214 ein Prägemuster 201 aufweist. An den Seiten des Deckels 214, außerhalb des Prägemusters 201 sind erste Passungen 218a in Form von Haltelöchern 218a in den Deckel 214 eingebracht. Durch die Haltelöcher 218a kann der Deckel 214 auf die Druckluftkammer 228 passgenau auflegt werden. In Figur 6 ist das Gegenstück zu dem Deckel 214 der Druckluftpresse 219 gezeigt, die Druckluftkammer 228. Die Druckluftkammer 228 weist zu den Haltelöchern 218a passend angebrachte zweite Passungen 218b, in Form von Halteelementen 218b auf. Weiterhin ist in der Druckluftkammer 228 ein ringförmiger Gummiring 230 eingebracht, der bei verschlossener Druckluftkammer 228 durch den Deckel 214, die Druckluftpresse 219 luftdicht hält, um den durch den Druckluftanschluss 232 eingeleiteten Luftdruck in der Druckluftkammer 228 halten zu können. Zur Einprägung einer Prägung 202 in eine Aluminiumfolie 100, 106 wird die Aluminiumfolie 100, 106 auf die Druckluftkammer 228 positioniert und, die Druckluftkammer 228 mit dem Deckel 214 verschlossen, sodass die Aluminiumfolie 100, 106 zwischen dem Deckel 214 und dem Gummiring 230 bzw. dem Boden der Druckluftkammer 228 liegt. Durch Einleiten von Gas durch den Druckluftanschluss 232 und den Lufteinlass 236 der Kammer 228 in die Druckluftkammer 228, zum Beispiel in Form von Luft, wird ein Druck auf die Aluminiumfolie 100, 106 erzeugt, der die Aluminiumfolie 100, 106 auf das Prägemuster 201 des Deckels 214 drückt. Eine Entlüftung 218 in dem Deckel 214 sorgt dafür, dass die eingepresste Luft über den Deckel 214 aus der Druckluftkammer 228 entweichen kann. Das Material der Druckluftpresse 219 sollte so gewählt werden, dass es dem Überdruck während des Prägens standhält. Beispielsweise kann der Deckel 214 und die Kammer 228 aus Stahl oder Aluminium gefertigt sein, wie in diesem Beispiel.

Figur 1b zeigt den Vorgang des Pressens einer vorgeprägten Aluminiumfolie 107. Dies kann eine Aluminiumfolie 100 sein oder ein Aluminium/Polymerverbund 106. In diesem Beispiel ist es ein Aluminium/Polymerverbund 106. Eine Aluminiumfolie 100 wurde hierzu mit einem Polyester (20 % Vitel^{®} 2200B-Lösung in Ethyl-Methyl-Keton der Firma Bostik, mit 10 bis 25 Gew.-% MoS₂) berakelt und anschließend bei 100°C für 5 Minuten getrocknet. Die Trocknung kann auch bei tieferen oder höheren Temperaturen, bevorzugt zwischen 20 und 200°C vorgenommen werden. Als Rakel kann beispielsweise, wie hier, ein Stahl-Rakel-Lineal aus V2a-Stahl mit einer Dimensionierung von 1*5*30 cm über einen Granittisch verwendet werden. Die Polymerschicht 104 kann alternativ über irgendein, dem Fachmann bekanntes Verfahren, wie Sprühen, Rollen, Pinseln, Abklatschen, Drahtrakeln, Rollrakeln, Siebdrucken, Tauchen, Gravurwalzen etc. aufgebracht werden. Auf diese Weise entstand eine Aluminiumfolie mit einer 4 µm dicken Polymerschicht 104. Bei dem in Fig. 1b schematisch gezeigten Pressvorgang wird die Aluminiumfolie 100 oder der Aluminium/Polymerverbund 106 zwischen einen Stempel 200 und eine Pressform 204 gepresst. Die Pressform kann dabei in Form eines Behälters oder in Form eines Ringes vorliegen. Da nur der Bereich mit den Prägungen 202 von dem Stempel 200 in die Press-form 204 gepresst werden kann das Innere der Aluminiumfolie 100 bzw. Aluminium/Polymerverbund 106 ohne Prägemuster 201 ausgestaltet sein. Bei dem Pressvorgang bewegt sich der Stempel 200 in Richtung des Pfeils 206. Dabei berührt der Stempel 200 bzw. das gefräste Bauteil 212 eines Prägewerkzeugs 210, aus Figur 2a, mindestens einen Teil der Prägungen 202 des Prägemusters 201 auf der Aluminiumfolie 100 bzw. 106. Zusammen mit dem Stempel 200 wird die Folie 100 bzw. der Verbund 106 in die Pressform 204 gepresst. Die Folie 100 bzw. der Verbund 106 werden dabei durch den Spalt 208, der sich zwischen Stempel 200 und Passform 204 ausbildet, gepresst. Dieser Spalt 208 wird auch als Spaltmaß 208 bezeichnet. Abhängig von dem Spaltmaß 208 kann die Aluminiumfolie 100 bzw. der Verbund 106 unterschiedlich tief in das Prägewerkzeug 210 gepresst werden. Zusätzlich hängt die Belastbarkeit von der Dicke der Aluminiumfolie 100 bzw. dem Verbund 106, die im Folgeneden nur noch als Folie 100, 106 bezeichnet wird, sowie der Form und Tiefe des Prägemusters 201 ab.

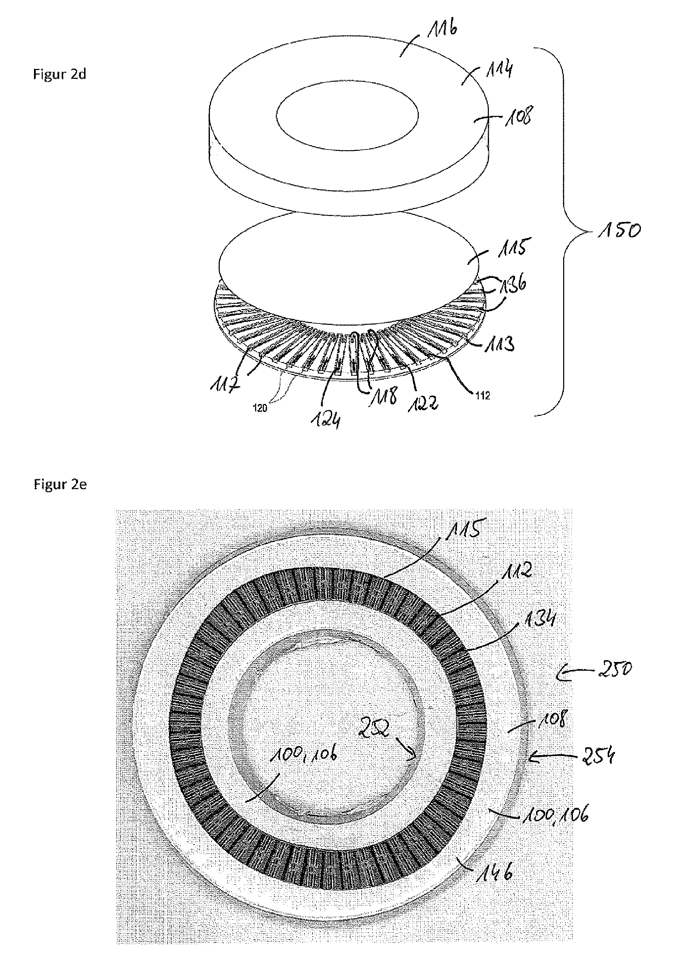

Ein Beispiel eines runden Stempels 200 und einer entsprechenden Pressform 204 ist in Figur 2b gezeigt. Figur 2c zeigt ein Beispiel für die Verwandlung einer unbehandelten Aluminiumfolie 100, 220 in Form einer kreisförmigen unbehandelten Aluminiumfolie 220 zu einer geprägten Aluminiumfolie 222 hin zu einer tiefgepressten Aluminiumfolie 224. Diese unbehandelte Aluminiumfolie 220 die zu Beginn weder geprägt noch gepresst ist, weist als geprägte Folie 222 ein Prägemuster 201 auf. Dieses Prägemuster 201 wird in dem Pressvorgang, wie für Figur 1b bereits beschrieben aus der Folie 220 vollständig gepresst. Nach dem Pressen ist die Aluminiumfolie 100, 224 eine Aluminiumfolie mit Vertiefung 140 in Form eines Behälters 146. Dieser Behälter 146 besitzt einen Boden 144 der in seiner Form der Pressform 204 entspricht. Die Presstiefe 142 liegt in diesem Beispiel bei ca. 3 mm. Der Boden 144 der Aluminiumfolie mit Vertiefung 140 kann weiter bearbeitet werden, in dem er beispielsweise ausgeschnitten wird. Hierdurch kann er der Form einer Fassung 113, wie in Figur 2d gezeigt, angepasst werden. Desweiteren kann eine Pressform 204 und ein Stempel 200 benutzt werden, der eine andere Form als die in Figur 2c gezeigte aufweist, sodass mehr als eine Vertiefung in die Aluminiumfolie 100, 106 eingebracht werden kann.

In Figur 2d ist gezeigt, wie die Aluminiumfolie mit Vertiefung 140 dazu verwendet wird, eine Fassung 113 mit mindestens einem analytischen Hilfsmittel 112 zumindest zu einem Teil zu bedecken. Die Aluminiumfolie 100, 106 kann dabei eine weitere Polymerschicht 114 aufweisen. Die Fassung 113 kann dabei aus einem Stück geformt sein, oder wie hier gezeigt, aus zwei Elementen 113 und 115 bestehen. Neben der Fassung 113 in der die Ausnehmungen 134, mit mindestens einer Wandung 136, für die analytischen Hilfsmittel 112 eingebracht sind, kann noch ein Fassungsdeckel 115 vorgesehen sein, der beispielsweise mit der Fassung über ein Laserschweißverfahren verbunden wird. Die beiden Elemente 113 und 115 können auch über einen anderen Prozess miteinander verbunden werden, wie beispielsweise einem Klebeprozess. Auf diese Weise wird bewirkt, dass nur noch wenige Öffnungen 117 und 118 in der Fassung 113 vorhanden sind, die verschlossen werden müssen, um die in den Ausnehmungen 134 befindlichen analytischen Hilfsmittel 112 sterildicht zu verschließen. Hierbei wird mindestens eine vorhandene Öffnung 117 und optional eine zweite Öffnung 118 der Fassung 113 bedeckt. Die Aluminiumfolie 100, 106 kann bei dem Abdecken der Öffnungen 117, 118 einem Zug ausgesetzt werden, sodass die Aluminiumfolie 100, 106 anschließend wieder eine nahezu glatte Oberfläche aufweist. Hierbei können restliche Prägungen 202, die durch den Pressvorgang aus Figur 1b nicht gänzlich entfernt wurden, glatt gezogen werden. Nach dem Bedecken der Öffnungen 117 und 118 mit der Aluminiumfolie wird die Aluminiumfolie über ein Heißsiegelverfahren mit der Fassung 113 zu dem Behälter 146 verschmolzen. Auf diese Weise können die analytischen Hilfsmittel 112 hermetisch in der Verpackung 250 eingeschlossen werden.

Das analytische Hilfsmittel 112 aus Figur 2d weist mindestens ein Stechelement 120 oder ein Testfeld 122 auf, oder beides. In dem gezeigten Beispiel in Figur 2d weist das kreisförmige analytische Hilfsmittel 112 mehrere Stechelemente 120, in Form von Lanzetten 120, und mehrere Testfelder 122 auf. In diesem Beispiel sind die Testfelder 122 und Lanzetten 120 jeweils zusammen in mehreren Ausnehmungen 134 untergebracht. In einer bevorzugten Ausführungsform sind jeweils eine Lanzette 120 und ein Testfeld 122 so zueinander angeordnet, dass eine Flüssigkeit, die an der Lanzette 120 anhaftet auf das Testfeld 122 übertragbar ist. Dies kann, wie in diesem Beispiel dadurch bewerkstelligt werden, dass die Lanzette 120 und das Testfeld 122 übereinander angeordnet sind, sodass durch Ausüben eines leichten Drucks auf Lanzette 120 oder Testfeld 122 die Flüssigkeit, in diesem Beispiel das Blut, auf das Testfeld 122 übertragen werden kann. In einer alternativen Ausführungsform, die hier nicht gezeigt ist, sind die Lanzette 120 und das Testfeld 122 über eine Kapillare mit einer hydrophilen Beschichtung 124 verbunden. Es handelt sich in diesem Beispiel um einen Microsampler als analytisches Hilfsmittel 112. Die analytischen Hilfsmittel 112 sind bevorzugt in allen beschriebenen Ausführungsformen in einer ringförmigen Fassung 113 mit eingelassenen Ausnehmungen 134 zusammengehalten.

In Figur 2e ist ein fertiger kreisförmiger Behälter 146 in Form einer Verpackung 250 gezeigt, der sowohl auf der Kreis Innenseite 252 als auch an der Kreis Außenseite 254 eine Aluminiumfolie 100, 106 aufweist. Zwischen den beiden Aluminiumfolien 100, 106 sind die kreisförmig angeordneten analytischen Hilfsmittel 112 zu erkennen, die in den Ausnehmungen 134 eingebracht sind. Ein Teil der analytischen Hilfsmittel 112 ist von den Aluminiumfolien 100, 106 abgedeckt. Auf der Innenseite 252 der Fassung 113 ist bevorzugt eine gepresste Aluminiumfolie 100, 106 über die Öffnung 118 gespannt. Beide Aluminiumfolien 100, 106 wurden über ein Heißsiegelverfahren mit der Fassung 113 verschmolzen. Hierzu ist mindestens eine Polymerschicht 104 auf der Aluminiumfolie 106 in Richtung zur Fassung 113 angeordnet, um bei Erhitzen mit dem Material der Fassung 113 verschmelzen zu können. Das Heißsiegeln kann, wie in diesem Beispiel bei 200 bis 210 °C und 150 bis 160 bar, der sich über die Folie verteilt, für eine Dauer von 0,1 bis 1,5 Sekunden durchgeführt werden.

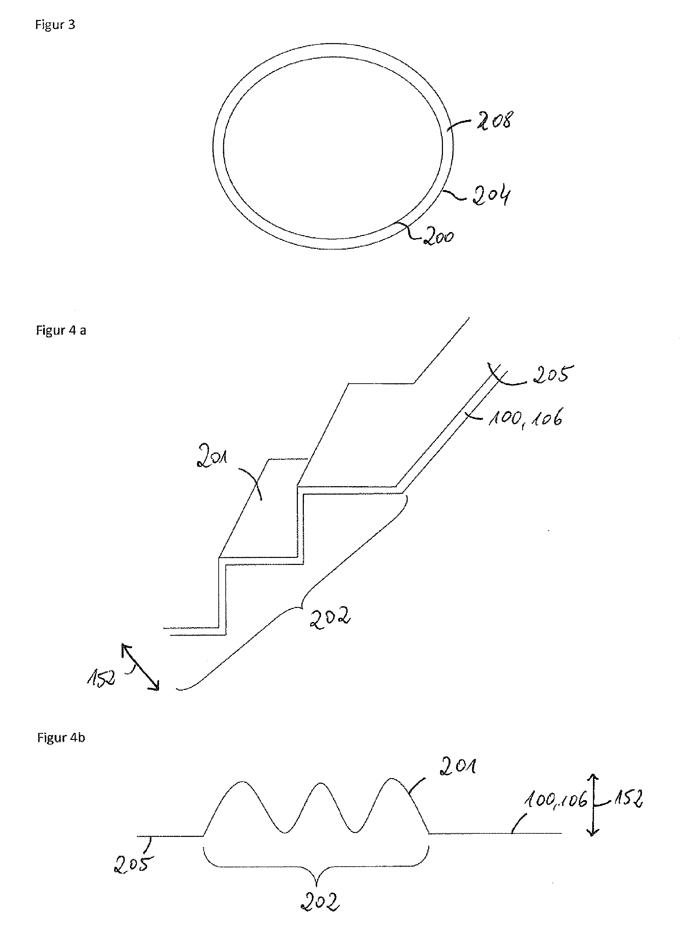

In Figur 4a und 4b ist ein Prägemuster 201 auf einer Aluminiumfolie 100 gezeigt, das Prägungen 202 aufweist. Es können auch mehr oder weniger Prägungen 202 vorhanden sein als in der Figur 4a gezeigt. Neben dem zackenartigen Prägemuster 201 in Figur 4a kann das Prägemuster 201 auch beispielsweise wellenförmig ausgestaltet sein, wie in Figur 4b gezeigt. Durch dieses Prägemuster 201 erhält die Aluminiumfolie 100 einen anderen Maximalquerschnitt 152 als die ungeprägte Aluminiumfolie 100 aus Figur 1a, da durch das Prägen Verwerfungen entstehen. Die Dicke der Folie 100 bleibt dabei bevorzugterweise konstant.

**Bezugszeichenliste**

| | | | |
|---|---|---|---|
| 100 | Aluminiumfolie | 205 | flächige Aluminiumfolie |
| 101 | Erste Seite | 206 | Pressrichtung |
| 102 | Zweite Seite | 208 | Spalt/Spaltmaß |
| 104 | Erste Polymerschicht-, folie | 210 | Prägewerkzeug |
| 106 | Aluminium/Polymerverbund | 212 | Gefrästes Bauteil |
| 107 | Vorgeprägte Folie | 214 | Deckel |
| 108 | Wachsschicht | 216 | Schaumstoff |
| 112 | Analytisches Hilfsmittel | 218 | Entlüftung |
| 113 | Fassung | 218a | Halteloch / erste Passung |
| 114 | Weitere Polymerschicht / Schutzlack | 218b | Halteelement / zweite Passung |
| 115 | Fassungsdeckel | 219 | Druckluftpresse |
| 116 | Verpackungsbestandteil | 220 | Unbehandelte Aluminiumfolie |
| 117 | Öffnung | 222 | Geprägte Aluminiumfolie |
| 118 | Zweite Öffnung | 224 | Tiefgepresste Aluminiumfolie |
| 120 | Stechelement, Lanzette | 228 | Druckluftkammer |
| 122 | Testfeld | 230 | Gummiring |
| 124 | Hydrophile Beschichtung | 232 | Druckluftanschluss |
| 134 | Ausnehmung | 234 | Boden der Druckluftkammer |
| 136 | Wandung | 236 | Lufteinlass |
| 138 | Vertiefung | 250 | Verpackung |
| 140 | Aluminiumfolie mit Vertiefung | 252 | Kreis Innenseite |
| 142 | Presstiefe | 254 | Kreis Außenseite |
| 144 | Boden der Vertiefung | | |
| 146 | Behälter | | |
| 148 | flächiges Verbundmaterial | | |
| 150 | Verpackung | | |
| 152 | Maximalquerschnitt | | |
| 200 | Stempel | | |
| 201 | Prägemuster | | |
| 202 | Prägung | | |
| 203 | Kreisförmige Fläche | | |
| 204 | Pressform | | |

## Patentansprüche

1. Ein Behälter (146) zumindest teilweise gebildet aus einem flächigen Verbundmaterial (148), wobei das Verbundmaterial (148) beinhaltet:
- eine Aluminiumfolie (100, 106) mit einer ersten (101) und einer zweiten Oberflächenseite (102),
- eine erste Polymerschicht (104), die mit mindestens einer der beiden Oberflächenseiten (101, 102) verbunden ist,
wobei die Aluminiumfolie (100, 106) über die Polymerschicht (104) mindestens eine Öffnung (117, 118) einer Fassung (113) bedeckt, wobei das Verbundmaterial (148) zusammen mit der Fassung (113) den Behälter (146) bilden, wobei die Fassung (113) mindestens ein analytisches Hilfsmittel (112) in einer Ausnehmung (134) aufnimmt, wobei die Aluminiumfolie gepresst oder tiefgezogen ist.

2. Der Behälter (146) nach Anspruch 1, wobei die Polymerschicht (104) aus einem thermoplastischen Polymer besteht.

3. Der Behälter (146) nach Anspruch 2, wobei das thermoplastische Polymer ein Polyester ist.

4. Der Behälter (146) nach Anspruch 3, wobei das Polyester ausgewählt ist aus der Gruppe bestehend aus: Polycarbonat, Polyethylennaphthalat, Polybutylentherephthalat, Polyethylentherephthalat und Polyesterharz oder einer Mischung aus mindestens zwei davon.

5. Der Behälter (146) nach einem der vorherigen Ansprüche, wobei die Aluminiumfolie (100, 106) eine Dicke zwischen 10 und 30 µm aufweist.

6. Der Behälter (146) nach einem der vorherigen Ansprüche, wobei das Verbundmaterial (148) eine weitere Polymerschicht (114) beinhaltet.

7. Der Behälter (146) nach einem der vorherigen Ansprüche, wobei das mindestens eine analytische Hilfsmittel (112) ein Stechelement (120) oder ein Testfeld (122) oder beides zum Nachweis eines Analyten in einer Körperflüssigkeit aufweist.

8. Der Behälter (146) nach einem der vorherigen Ansprüche, wobei das analytische Hilfsmittel (112) eine hydrophile Beschichtung (124) aufweist.

9. Der Behälter (146) nach einem der vorherigen Ansprüche, wobei die erste Polymerschicht (104) so ausgestaltet ist, dass das analytische Hilfsmittel (112) vor Gebrauch hydrophil gehalten wird.

10. Der Behälter (146) nach einem der vorherigen Ansprüche, wobei die erste Polymerschicht (104) eine Dicke in einem Bereich von 0,5 bis 10 µm, bevorzugt in einem Bereich von 2 bis 8 µm und besonders bevorzugt in einem Bereich von 3 bis 6 µm aufweist.

11. Der Behälter (146) nach einem der vorherigen Ansprüche, wobei der Behälter (146) erhältlich ist aus einer Aluminiumfolie (100, 106) mit mindestens einer Prägung (202).

12. Ein Verfahren zur Herstellung eines Behälters (146) zur Bevorratung von analytischen Hilfsmitteln (112), beinhaltende die Schritte:
- Bereitstellen einer Fassung (113) mit einer Vielzahl analytischer Hilfsmittel (112),
- Abdecken mindestens eines Teils der Fassung (113) mit einem flächigen Verbundmaterial (148) beinhaltend eine Aluminiumfolie (100, 106) die mit einer Polymerschicht (104) verbunden ist, wobei die Polymerschicht (104) zu der Fassung (113) weist,
- Erhitzen mindestens der Polymerschicht (104) sodass die Polymerschicht (104) mindestens teilweise aufschmilzt und mit der Fassung (113) eine Verbindung eingeht und einen Behälter (146) bildet, wobei vor oder beim Abdecken mindestens eines Teils der Fassung mit dem flächigen Verbundmaterial, die Aluminiumfolie gepresst oder tiefgezogen wird.

13. Das Verfahren nach Anspruch 12, wobei die Polymerschicht (104) aus einem thermoplastischen Polymer besteht.

14. Das Verfahren nach Anspruch 13, wobei das thermoplastische Polymer ein Polyester ist.

15. Das Verfahren nach Anspruch 14, wobei das Polyester ausgewählt ist aus der Gruppe bestehend aus: Polycarbonat, Polyethylennaphthalat, Polybutylentherephthalat, Polyethylentherephthalat und Polyesterharz oder einer Mischung aus mindestens zwei davon.

## Claims

1. Container (146) at least to some extent composed of a sheet of composite material (148), where the composite material (148) includes:
- an aluminum foil (100, 106) with a first (101) and a second surface side (102),
- a first polymer layer (104), bonded to at least one of the two surface sides (101, 102), where the aluminum foil (100, 106) covers, by way of the polymer layer (104), at least one aperture (117, 118) of a holder (113), where the composite material (148) and the holder (113) together form the container (146), where the holder (113) accepts at least one analytical aid (112) in a cutout (134), where the aluminum foil has been pressed or deep-drawn.

2. Container (146) according to Claim 1, where the polymer layer (104) is composed of a thermoplastic polymer.

3. Container (146) according to Claim 2, where the thermoplastic polymer is a polyester.

4. Container (146) according to Claim 3, where the polyester is one selected from the group consisting of: polycarbonate, polyethylene naphthalate, polybutylene terephthalate, polyethylene terephthalate, and polyester resin, and mixtures of at least two thereof.

5. Container (146) according to any of the preceding claims, where the thickness of the aluminum foil (100, 106) is from 10 to 30 µm.

6. Container (146) according to any of the preceding claims, where the composite material (148) includes a further polymer layer (114).

7. Container (146) according to any of the preceding claims, where the at least one analytical aid (112) comprises a puncture element (120) or a test field (122) or both, for the detection of an analyte in a body fluid.

8. Container (146) according to any of the preceding claims, where the analytical aid (112) comprises a hydrophilic coating (124).

9. Container (146) according to any of the preceding claims, where the design of the first polymer layer (104) is such that the analytical aid (112) is kept hydrophilic prior to use.

10. Container (146) according to any of the preceding claims, where the thickness of the first polymer layer (104) is in the range from 0.5 to 10 µm, preferably in the range from 2 to 8 µm, and particularly preferably in the range from 3 to 6 µm.

11. Container (146) according to any of the preceding claims, where the container (146) is obtainable from an aluminum foil (100, 106) with at least one embossment (202).

12. Process for the production of a container (146) for the storage of analytical aids (112), including the following steps:
- provision of a holder (113) with a plurality of analytical aids (112),
- protective covering of at least one portion of the holder (113) with a sheet of composite material (148) including an aluminum foil (100, 106) bonded to a polymer layer (104), where the polymer layer (104) faces toward the holder (113),
- heating at least of the polymer layer (104), so that the polymer layer (104) melts at least to some extent and bonds to the holder (113) and forms a container (146), where, prior to or during the process of protective covering of at least one portion of the holder with the sheet of composite material, the aluminum foil is pressed or deep-drawn.

13. Process according to Claim 12, where the polymer layer (104) is composed of a thermoplastic polymer.

14. Process according to Claim 13, where the thermoplastic polymer is a polyester.

15. Process according to Claim 14, where the polyester is one selected from the group consisting of: polycarbonate, polyethylene naphthalate, polybutylene terephthalate, polyethylene terephthalate, and polyester resin, and mixtures of at least two thereof.

## Revendications

1. Récipient (146) formé au moins en partie à partir d'un matériau composite plat (148), le matériau composite (148) contenant :
- une feuille d'aluminium (100, 106) comprenant un premier (101) et un deuxième côté superficiel (102) ;
- une première couche polymère (104) qui est reliée à au moins un des deux côtés superficiels (101, 102) ;
la feuille d'aluminium (100, 106) recouvrant par-dessus la couche polymère (104) au moins une ouverture (117, 118) d'un support (113), le matériau composite (148) formant le récipient (146) conjointement avec le support (113), le support (113) recevant au moins un adjuvant analytique (112) dans un évidement (134), la feuille d'aluminium étant comprimée ou emboutie.

2. Récipient (146) selon la revendication 1, dans lequel la couche polymère (104) est constituée d'un polymère thermoplastique.

3. Récipient (146) selon la revendication 2, dans lequel le polymère thermoplastique est un polyester.

4. Récipient (146) selon la revendication 3, dans lequel le polyester est choisi parmi le groupe constitué par un polycarbonate, un polyéthylènenaphtalate, un polybutylènetéréphtalate, un polyéthylènetéréphtalate et une résine de polyester ou un mélange d'au moins deux des éléments que l'on vient de citer.

5. Récipient (146) selon l'une quelconque des revendications précédentes, dans lequel la feuille d'aluminium (100, 106) présente une épaisseur entre 10 et 30 µm.

6. Récipient (146) selon l'une quelconque des revendications précédentes, dans lequel le matériau composite (148) contient une couche polymère supplémentaire (114).

7. Récipient (146) selon l'une quelconque des revendications précédentes, dans lequel ledit au moins un adjuvant analytique (112) présente un élément utilisé pour piquer (120) ou un champ de test (122) ou les deux pour le décèlement d'un analyte dans un liquide corporel.

8. Récipient (146) selon l'une quelconque des revendications précédentes, dans lequel l'adjuvant analytique (112) présente un revêtement hydrophile (124).

9. Récipient (146) selon l'une quelconque des revendications précédentes, dans lequel la première couche polymère (104) est réalisée de façon à maintenir le caractère hydrophile de l'adjuvant analytique (112) avant l'emploi.

10. Récipient (146) selon l'une quelconque des revendications précédentes, dans lequel la première couche polymère (104) présente une épaisseur dans la plage de 0,5 à 10 µm, de préférence dans la plage de 2 à 8 µm et de manière particulièrement préférée dans la plage de 3 à 6 µm.

11. Récipient (146) selon l'une quelconque des revendications précédentes, dans lequel on obtient le récipient (146) à partir d'une feuille d'aluminium (100, 106) comprenant au moins une empreinte (202).

12. Procédé pour la production d'un récipient (146) pour l'approvisionnement d'adjuvants analytiques (112), comprenant les étapes dans lesquelles :
- on procure un support (113) comprenant plusieurs adjuvants analytiques (112) ;
- on recouvre au moins une partie du support (113) avec un matériau composite plat (148) comprenant une feuille d'aluminium (100, 106) qui est reliée à une couche polymère (104), la couche polymère (104) étant orientée en direction du support (113) ;
- on chauffe au moins la couche polymère (104) de telle sorte que la couche polymère (104) entre au moins en partie en fusion et vient se relier au support (113) pour former un récipient (146), dans lequel, avant ou pendant le recouvrement d'au moins une partie du support avec le matériau composite plat, la feuille d'aluminium est comprimée ou emboutie.

13. Procédé selon la revendication 12, dans lequel la couche polymère (104) est constituée d'un polymère thermoplastique.

14. Procédé selon la revendication 13, dans lequel le polymère thermoplastique est un polyester.

15. Procédé selon la revendication 14, dans lequel le polyester est choisi parmi le groupe constitué par un polycarbonate, un polyéthylènenaphtalate, un polybutylènetéréphtalate, un polyéthylènetéréphtalate et une résine de polyester ou un mélange d'au moins deux des éléments que l'on vient de citer.
